(19) Europäisches Patentamt
European Patent Office
Office européen des brevets

(11) EP 2 915 796 A1

(12) # EUROPEAN PATENT APPLICATION
published in accordance with Art. 153(4) EPC

(43) Date of publication:
**09.09.2015 Bulletin 2015/37**

(21) Application number: **13850900.5**

(22) Date of filing: **31.10.2013**

(51) Int Cl.:
***C07C 43/23*** *(2006.01)* ***A01N 31/14*** *(2006.01)*
***A01P 3/00*** *(2006.01)* ***A61K 8/34*** *(2006.01)*
***A61K 8/37*** *(2006.01)* ***A61K 8/40*** *(2006.01)*
***A61K 8/41*** *(2006.01)* ***A61K 8/44*** *(2006.01)*
***A61K 8/46*** *(2006.01)* ***A61K 31/085*** *(2006.01)*
***A61K 31/10*** *(2006.01)* ***A61K 31/136*** *(2006.01)*
***A61K 31/192*** *(2006.01)* ***A61K 31/198*** *(2006.01)*
***A61K 31/277*** *(2006.01)* ***A61P 17/00*** *(2006.01)*
***A61P 31/04*** *(2006.01)* ***A61P 43/00*** *(2006.01)*
***A61Q 19/00*** *(2006.01)* ***A61Q 19/02*** *(2006.01)*

(86) International application number:
**PCT/JP2013/079498**

(87) International publication number:
**WO 2014/069555 (08.05.2014 Gazette 2014/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.11.2012 JP 2012242916**

(71) Applicant: **Seiwa Kasei Company, Limited**
**Higashiosaka-shi**
**Osaka 579-8004 (JP)**

(72) Inventors:
- **YOSHIOKA, Masato**
  **Higashiosaka-shi**
  **Osaka 579-8004 (JP)**
- **TAIRA, Norihisa**
  **Higashiosaka-shi**
  **Osaka 579-8004 (JP)**
- **NAKAMURA, Sayaka**
  **Higashiosaka-shi**
  **Osaka 579-8004 (JP)**

(74) Representative: **Duckworth, Timothy John**
**J A Kemp**
**14 South Square**
**Gray's Inn**
**London WC1R 5JJ (GB)**

(54) **PROPYL-PHENYL-ETHER DERIVATIVE AND MELANOGENESIS INHIBITOR, SKIN-LIGHTENING AGENT, ANTIMICROBIAL AGENT, AND COSMETIC CONTAINING SAID PROPYL-PHENYL-ETHER DERIVATIVE**

(57) Provided is a compound that exhibits an excellent melanogenesis-inhibiting effect (skin-lightening effect), exhibits an excellent antimicrobial effect, excels in terms of temporal stability and the like, and is suitable for use as an ingredient of a cosmetic. Said compound is a propyl-phenyl-ether derivative compound comprising a propyl group that has a substituent such as a hydroxyl group and is bound to the hydroxyl group of a phenol group that has a substituent such as a tert-butyl group. A melanogenesis inhibitor, skin-lightening agent, and antimicrobial agent containing the aforementioned compound as an active ingredient are also provided, as is a cosmetic characterized by containing said compound.

EP 2 915 796 A1

## Description

(Technical Field)

The present invention relates to a propyl-phenyl-ether derivative which is suitably used as a raw material of a cosmetic, and the like. Further, the present invention relates to a melanogenesis inhibitor, a skin-lightening agent and an antimicrobial agent containing the above-described propyl-phenyl-ether derivative as an active ingredient, and to a cosmetic prepared by formulating the above-described propyl-phenyl-ether derivative.

(Background Art)

Recently, a cosmetic is often used expecting skin-lightening effects such as a melanogenesis inhibiting effect and the like, an antimicrobial effect, and various effects such as an anti-acne effect, an anti-dandruff effect, an anti-wrinkle effect and the like. As the raw material of a cosmetic, those performing the above-described effects are desired. There is a high expectation for skin-lightening effects, particularly a melanogenesis inhibiting effect, among others.

When skin is exposed to ultraviolet, active oxygen is generated in the skin and various substances are released from the surrounding cells. By the active oxygen and various substances, melanocyte is activated to enhance tyrosinase activity, thereby generating melanin excessively. This melanin is transferred to epidermal cell, and resultantly, the color tone of skin changes and skin is tanned. Further, when a balance between generation and discharge of melanin is lost and melanin is accumulated excessively in epidermal cell, spots and freckles are formed. Then, for preventing excess accumulation of melanin, there is a suggestion on various skin-lightening agents such as hydroquinone derivatives such as kojic acid, hydroquinone, arbutin and the like, vitamin C and derivatives thereof, and the like.

Kojic acid, arbutin and vitamin C and derivatives thereof show an effect of suppressing generation of melanin and preventing tanning, however, its effect is insufficient. Further, the majority of kojic acid, vitamin C and derivatives thereof, when formulated in a cosmetic, cause a problem of coloration with time.

Hydroquinone is problematic in safety and stability, and said to be difficult to use in a cosmetic. For improving this problem, various hydroquinone derivatives are suggested. For example, while hydroquinone alkyl ethers and the like are suggested in patent document 1, these compounds have hydroquinone as a mother skeleton and safeness thereof is a concern.

There are also suggestions on an acylated derivative of 2-(4-hydroxyphenyl)ethanol having a phenolic hydroxyl group (patent document 2) and an alkyl phenyl ether glycoside (patent document 3). However, these derivatives are believed to have generally poor stability since a phenolic hydroxyl group is bound by an ester bond and a glycosidic bond, and there is a concern about safety since a phenolic hydroxyl group is liberated by hydrolysis.

As the compound having a high melanogenesis inhibiting effect, alkylphenols such as tert-butylphenol and the like are also known. However, these compounds are not good in safety and cannot be applied to a cosmetic (non-patent document 1).

On the other hand, in the cosmetics market, new antimicrobial compounds are also required for antisepsis of cosmetic product formulations, and further, for suppression of activity of propionibacterium acnes as one cause of acne, malassezia bacterium as a cause of scalp dandruff and itching, a microorganism causing underarm odor, and the like. Then, tocopheryl phosphate is proposed as one showing an antimicrobial action against propionibacterium acnes (patent document 4). This compound, however, shows an effect only against propionibacterium acnes and does not exhibit wide antimicrobial effects.

(Prior Art Document)

(Patent Document)

(Patent document 1) JP-A No. 6-192062
(Patent document 2) Japanese Patent No. 4658898
(Patent document 3) Japanese Patent No. 3340930
(Patent document 4) Japanese Patent No. 4828126

(Non-Patent Document)

(Non-Patent document 1) Journal of Investigative Dermatology, Vol.114(1),pp157-164(2000)

(Summary of the Invention)

(Problem to be Solved by the Invention)

Recently, the requirement for a cosmetic is further increased, and there is a desire for development of a cosmetic having more excellent skin-lightening effects such has a melanogenesis inhibiting effect and the like or/and an antimicrobial effect and is excellent in temporal stability and the like. Then, an object of the present invention is to provide a compound having an excellent melanogenesis inhibiting effect (skin-lightening effect) and an antimicrobial effect and which is excellent also in temporal stability and the like and used suitably as a raw material of a cosmetic.

Also, an object of the present invention is to provide a melanogenesis inhibitor or a skin-lightening agent which contains the above-described compound as an active ingredient, prevents tanning of skin, and improves pigment depositions such as spots and freckles and the like.

Also, an object of the present invention is to provide an antimicrobial agent containing the above-described compound as an active ingredient and which can prevent bacterial contamination of a cosmetic product, acne and dandruff.

Further, an object of the present invention is to provide a cosmetic prepared by formulating the above-described compound, especially, a skin-lightening cosmetic.

(Means for Solving the Problem)

The present inventors have intensively studied in view of the above-described circumstances and resultantly found that a propyl-phenyl-ether derivative compound substituted with a specific functional group and its salt have an excellent melanogenesis inhibiting effect and/or an antimicrobial effect and are excellent in stability. Thus, the present invention was completed.

The present invention provides a propyl-phenyl-ether derivative represented by the following general formula (I), (Ia) or (Ib) (Claim 1).

(Chemical formula 1)

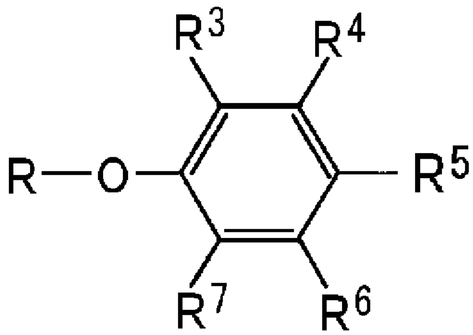

(I)

(Chemical formula 2)

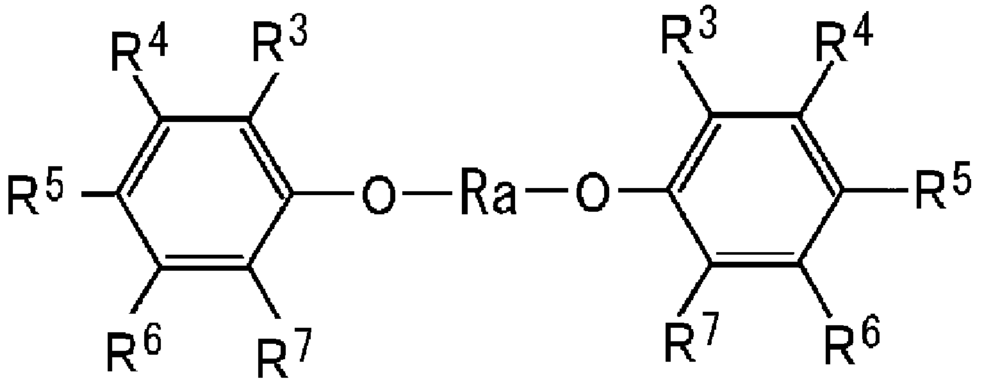

(Ia)

(Chemical formula 3)

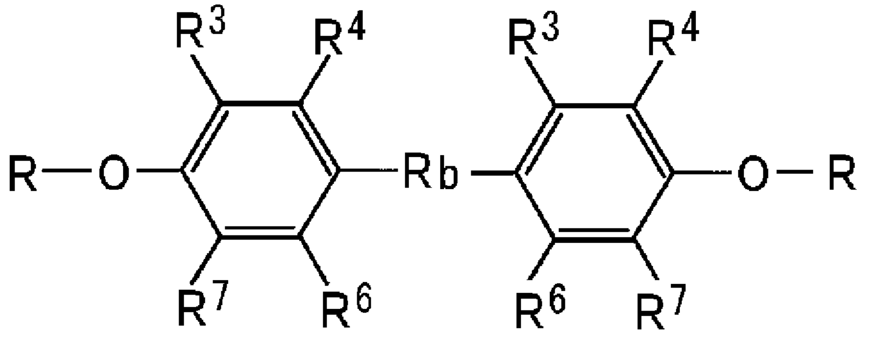

(Ib)

In the formula (I), (Ia) or (Ib),
R represents $-CH_2-CHR^2-CH_2R^1$ or $-CH(CH_2R^2)-CH_2R^1$,
Ra represents $-CH_2-CHR^2-CH_2-O-CH_2-CHR^2-CH_2-$ or $-CH_2-CHOH-CH_2$ -,
Rb represents a linear or branched aliphatic hydrocarbon group having 1 to 5 carbon atoms,
$R^1$ and $R^2$ represent each independently hydrogen, $R^8O$, $R^9S$, $R^{10}R^{11}N$, $R^{12}A1$, a linear or branched, saturated or unsaturated aliphatic hydrocarbon group having 1 to 22 carbon atoms, or a saturated or unsaturated cyclic hydrocarbon group having 3 to 22 carbon atoms,
$R^8$, $R^9$, $R^{10}$, $R^{11}$ and $R^{12}$ represent each independently a linear or branched, saturated or unsaturated aliphatic hydrocarbon group having 1 to 22 carbon atoms, a saturated or unsaturated cyclic hydrocarbon group having 3 to 22 carbon atoms of which hydrogen may be substituted with a hydroxyl group, or hydrogen,
A1 represents a phosphate group, a sulfate group, an ester group, a thioester group or an amide group or a salt thereof, and here,
at least one of $R^1$ or $R^2$ is a hydroxyl group, or a group represented by $R^{12}A1$- in which A is a phosphate group, a sulfate group or an ester group or a salt thereof, and
$R^3$, $R^4$, $R^5$, $R^6$ and $R^7$ represent each independently
a linear or branched, saturated or unsaturated aliphatic hydrocarbon group having 1 to 12 carbon atoms of which hydrogen may be substituted with a phenyl group, an alkoxy group having 1 to 5 carbon atoms, a phenyl group, or hydrogen,
and when both $R^1$ and $R^2$ are a group selected from the group consisting of a hydroxyl group and a group represented by $R^{12}A1$- in which A is a phosphate group, a sulfate group or an ester group or a salt thereof, the sum of the number of carbon atoms of $R^3$, $R^4$, $R^5$, $R^6$ and $R^7$ is 2 or more.

When R in the formula (I) is $-CH_2-CHR^2-CH^2R^1$ or is-$CH(CH_2R^2)-CH_2R^1$, the propyl-phenyl-ether derivative compound of the formula (I) is represented by the structural formula (Ic) or the structural formula (Id) described below, respectively.

(Chemical formula 4)

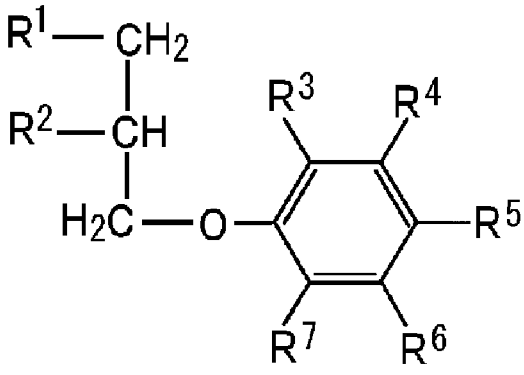

(Ic)

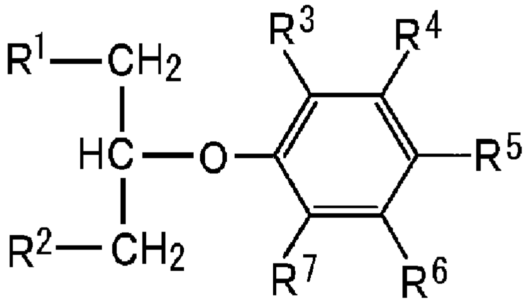

(Id)

$R^1$ and $R^2$ and $R^8$, $R^9$, $R^{10}$, $R^{11}$ and $R^{12}$ may be an aliphatic hydrocarbon having 1 to 22 carbon atoms or a cyclic hydrocarbon having 3 to 22 carbon atoms. Here, the aliphatic hydrocarbon having 1 to 22 carbon atoms may be any of linear or branched, and in both cases of linear and branched, may be any of a saturated or unsaturated hydrocarbon. Further, the cyclic hydrocarbon having 3 to 22 carbon atoms denotes a hydrocarbon having a saturated or unsaturated ring, and the unsaturated ring includes also aromatic rings. In the cyclic hydrocarbon having 3 to 22 carbon atoms, its hydrogen may be substituted with a hydroxyl group.

$R^3$, $R^4$, $R^5$, $R^6$ and $R^7$ may be an aliphatic hydrocarbon having 1 to 12 carbon atoms. Here, the aliphatic hydrocarbon having 1 to 12 carbon atoms may be linear or branched, and in both cases of linear and branched, may be a saturated hydrocarbon or may be an unsaturated hydrocarbon.

$R^1$ or $R^2$ may be a functional group represented by $R^{12}A1$, and here, the phosphate group, the sulfate group, the ester group, the thioester group and the amide group represented by A are divalent groups represented by the following structural formulae, respectively. In the formulae, * represents a group linking to $R^{12}$.

(Chemical formula 5)

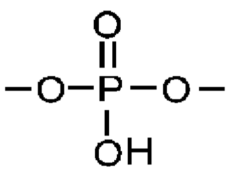

phosphate group

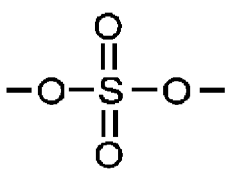

sulfate group

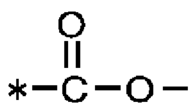

ester group

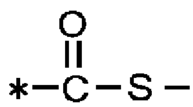

thioester group

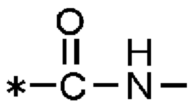

amide group

Among propyl-phenyl-ether derivatives as the propyl-phenyl-ether derivative compound and its salt represented by the general formula (I), (Ia) or (Ib), propyl-phenyl-ether derivatives in which $R^1$ or/and $R^2$ are a group selected from hydrogen, a linear or branched, saturated or unsaturated aliphatic hydrocarbon group having 1 to 22 carbon atoms, $R^8O$, $R^9S$ and $R^{10}R^{11}N$ are preferable since these show a more excellent melanogenesis inhibiting effect. Of them, cases in which $R^1$ is $R^8O$ and $R^2$ is a hydroxyl group are more preferable.

Then, the present invention provides a propyl-phenyl-ether derivative represented by the general formula (I), (Ia) or (Ib) in which $R^1$ or/and $R^2$ are a group selected from hydrogen, a linear or branched, saturated or unsaturated

aliphatic hydrocarbon group having 1 to 22 carbon atoms, $R^8O$, $R^9S$ and $R^{10}R^{11}N$ (Claim 2), and a propyl-phenyl-ether derivative in which $R^1$ is $R^8O$ and $R^2$ is a hydroxyl group (Claim 3), as preferable embodiments thereof.

Of the propyl-phenyl-ether derivatives according to the above-described preferable embodiments, those represented by the general formula (I) show a further excellent melanogenesis inhibiting effect. Of them, those in which $R^1$ is $R^8O$ and $R^2$ is a hydroxyl group are preferable. Then, the present invention provides a propyl-phenyl-ether derivative which is represented by the general formula (I) in which $R^1$ is $R^8O$ and $R^2$ is a hydroxyl group (Claim 4), as a preferable embodiment thereof.

Of these propyl-phenyl-ether derivatives, one in which $R^8$ is hydrogen or a linear or branched, saturated or unsaturated aliphatic hydrocarbon group having 1 to 22 carbon atoms (Claim 5) is particularly preferable, and especially, one in which $R^8$ is a linear or branched, saturated or unsaturated aliphatic hydrocarbon group having 1 to 22 carbon atoms (Claim 6) is preferable.

One in which $R^2$ is represented by $R^{12}A1$ and A is a phosphate group, a sulfate group or an ester group or its salt is easily hydrolyzed by phosphatase, esterase and the like in a living body to generate a hydroxyl group, and becomes a propyl-phenyl-ether derivative showing a particularly excellent melanogenesis inhibiting effect. Therefore, also cases in which $R^2$ is represented by $R^{12}A1$ and A is a phosphate group, a sulfate group or an ester group or its salt are preferable since these show an excellent melanogenesis inhibiting effect.

In the case of the propyl-phenyl-ether derivative represented by the general formula (I), those in which $R^3$, $R^4$, $R^5$, $R^6$ and $R^7$ are hydrogen or a linear or branched, saturated or unsaturated aliphatic hydrocarbon having 1 to 12 carbon atoms are preferable since these show an excellent melanogenesis inhibiting effect. Of them, those in which at least one group among $R^3$, $R^4$, $R^5$, $R^6$ and $R^7$ is a branched aliphatic hydrocarbon and the sum of the number of carbon atoms of $R^3$, $R^4$, $R^5$, $R^6$ and $R^7$ is 4 or more are more preferable. Further preferable are cases in which at least one group among $R^3$, $R^4$, $R^5$, $R^6$ and $R^7$ is a tert-butyl group or a sec-butyl group.

Then, the present invention provides a propyl-phenyl-ether derivative represented by the general formula (I) in which $R^3$, $R^4$, $R^5$, $R^6$ and $R^7$ represent each independently hydrogen or a linear or branched, saturated or unsaturated aliphatic hydrocarbon having 1 to 12 carbon atoms (Claim 7), a propyl-phenyl-ether derivative represented by the general formula (I) in which at least one group among $R^3$, $R^4$, $R^5$, $R^6$ and $R^7$ is a branched aliphatic hydrocarbon and the sum of the number of carbon atoms of $R^3$, $R^4$, $R^5$, $R^6$ and $R^7$ is 4 or more (Claim 8), and a propyl-phenyl-ether derivative represented by the general formula (I) in which at least one group among $R^3$, $R^4$, $R^5$, $R^6$ and $R^7$ is a tert-butyl group or a sec-butyl group (Claim 9), as preferable embodiments thereof.

Of propyl-phenyl-ether derivatives as the propyl-phenyl-ether derivative compound represented by the general formula (Ia) and its salt, those in which Ra is $-CH_2-CH(OH)-CH_2-$ are preferable since these show an excellent melanogenesis inhibiting effect. Then, the present invention provides a propyl-phenyl-ether derivative represented by the general formula (Ia) in which Ra is $-CH_2-CH(OH)-CH_2-$ (Claim 10), as preferable embodiments thereof.

Among propyl-phenyl-ether derivatives represented by the general formula (Ib), propyl-phenyl-ether derivatives in which Rb is $-C(CH_3)_2-$ are preferable since these show an excellent melanogenesis inhibiting effect. Then, the present invention provides a propyl-phenyl-ether derivative represented by the general formula (Ib) in which Rb is$-C(CH_3)_2-$ (Claim 11), as preferable embodiments thereof.

Among the above-described propyl-phenyl-ether derivatives, compounds represented by formula (II), formula (III), formula (A), formula (C), formula (D), formula (E), formula (F), formula (G), formula (H), formula (P), formula (J) or formula (K) shown below can be listed as those showing a particularly excellent melanogenesis inhibiting effect and which can be easily produced.

In the structural formulae shown below, a carbon atom and a hydrogen atom linking to the carbon atom are omitted in some cases. For example, the groups at 1-position and 3-position of (a propyl group) in the formula (II) and the formula (III) are a $CH_2$ group, and the group at 2-position is a CH group. The groups at 1'-position and 4'-position of (a phenyl group) are a carbon atom, and the groups at 2'-position, 3'-position, 5'-position and 6'-position are a CH group. The group linking at 4'-position in the formula (II) is a tert-butyl group, and the group linking at 1-position is an ethoxy group.

(Chemical formula 6)

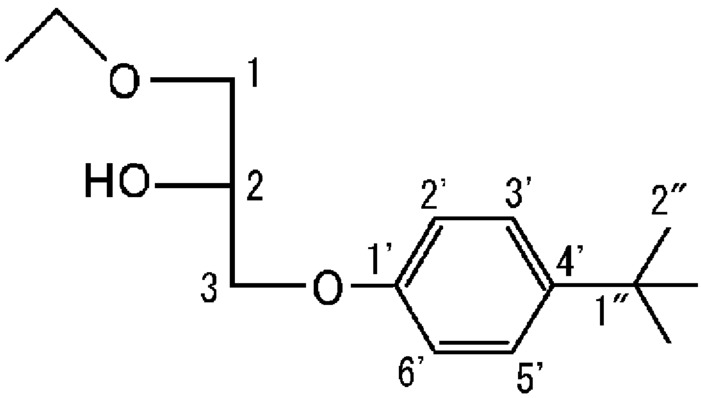

(II)

(Chemical formula 7)

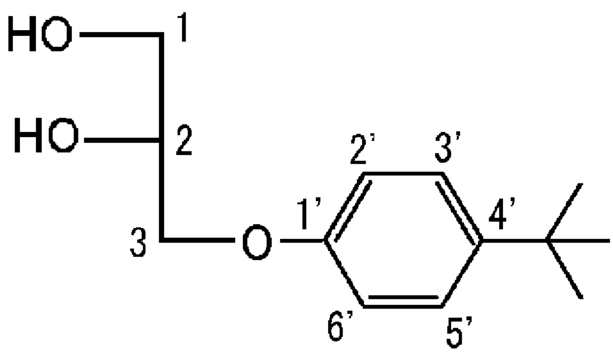

(III)

(Chemical formula 8)

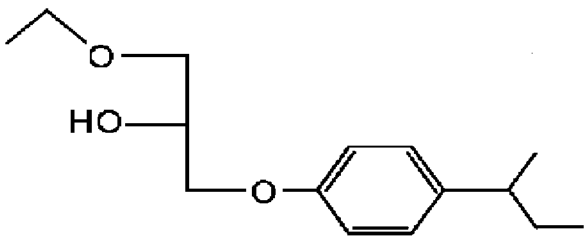

(A)

(Chemical formula 9)

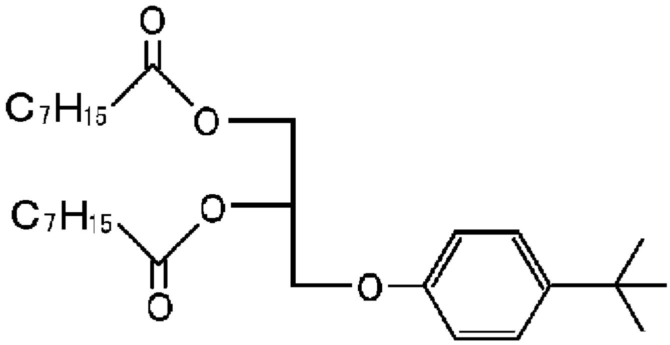

(C)

(Chemical formula 10)

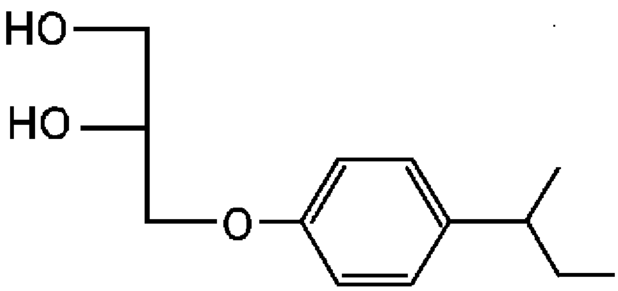

(D)

(Chemical formula 11)

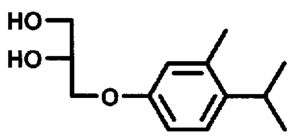

(E)

(Chemical formula 12)

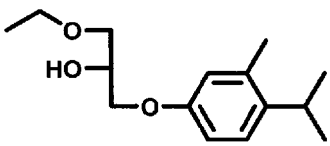

(F)

(Chemical formula 13)

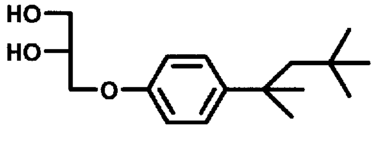

(G)

(Chemical formula 14)

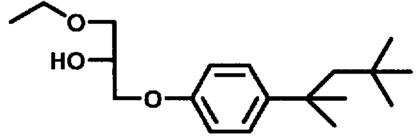

(H)

(Chemical formula 15)

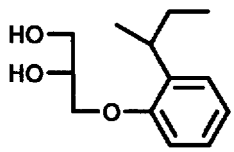

(P)

(Chemical formula 16)

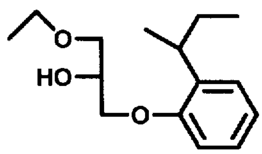

(J)

(Chemical formula 17)

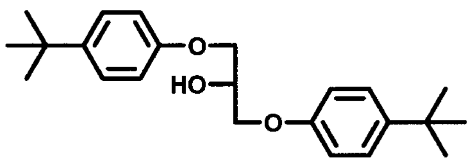

(K)

Among the above, the compounds represented by the structural formula (II), formula (A), formula (C), formula (E), formula (F), formula (G), formula (H), formula (P), formula (J) or formula (K) are novel compounds. Then, the present invention provides the following embodiments as novel compounds showing a particularly excellent melanogenesis inhibiting effect.

A propyl-phenyl-ether derivative represented by the structural formula (II)(Claim 12).

A propyl-phenyl-ether derivative represented by the structural formula (A) (Claim 13).

A propyl-phenyl-ether derivative represented by the structural formula (C)(Claim 14).

A propyl-phenyl-ether derivative represented by the structural formula (E) (Claim 15).

A propyl-phenyl-ether derivative represented by the structural formula (F) (Claim 16).

A propyl-phenyl-ether derivative represented by the structural formula (G)(Claim 17).

A propyl-phenyl-ether derivative represented by the structural formula (H) (Claim 18).

A propyl-phenyl-ether derivative represented by the structural formula (P) (Claim 19).

A propyl-phenyl-ether derivative represented by the structural formula (J) (Claim 20).

A propyl-phenyl-ether derivative represented by the structural formula (K) (Claim 21).

The propyl-phenyl-ether derivative represented by the general formula (I) of the present invention shows an excellent melanogenesis inhibiting effect, an excellent skin-lightening effect and an excellent antimicrobial effect and has excellent stability, thus, can be suitably formulated in a cosmetic (including skin external agent). Then, the present invention provides a melanogenesis inhibitor, a skin-lightening agent, an antimicrobial agent and a cosmetic shown below, in addition to the above-described propyl-phenyl-ether derivative.

That is, the present invention provides a melanogenesis inhibitor prepared by formulating the propyl-phenyl-ether derivative according to any one of Claims 1 to 21 as an active ingredient (Claim 22).

The propyl-phenyl-ether derivatives represented by the formula (III) or the formula (D) show an excellent melanogenesis inhibiting effect as described above. As specific embodiments of the above-described melanogenesis inhibitor, a melanogenesis inhibitor prepared by formulating the propyl-phenyl-ether derivative represented by the formula (III) as an active ingredient (Claim 23) and a melanogenesis inhibitor prepared by formulating the propyl-phenyl-ether derivative represented by the formula (D) as an active ingredient (Claim 24) are provided.

The present invention provides a skin-lightening agent prepared by formulating the propyl-phenyl-ether derivative according to any one of Claims 1 to 21 as an active ingredient (Claim 25).

Further, the present invention provides an antimicrobial agent prepared by formulating the propyl-phenyl-ether derivative according to any one of Claims 1 to 21 as an active ingredient (Claim 26).

Further, the present invention provides a cosmetic prepared by formulating the propyl-phenyl-ether derivative compound and its salt according to any one of Claims 1 to 21 as an active ingredient (Claim 27). The amount of the propyl-phenyl-ether derivative of the present invention into the cosmetic is preferably usually 0.0001 mass% to 10 mass%. When less than 0.001 mass%, the melanogenesis inhibiting effect and the antimicrobial effect according to the present invention often cannot be manifested sufficiently. In contrast, when over 10 mass%, the agent system is possibly broken and an effect corresponding to the amount cannot be attained in many cases.

Since the propyl-phenyl-ether derivative of the present invention shows an excellent skin-lightening effect, the cosmetic of the present invention described above is suitably used as a skin-lightening cosmetic intending skin-lightening. It is suitably used also as a cosmetic intending an antimicrobial effect.

(Effect of the invention)

The propyl-phenyl-ether derivative compound represented by the above-described general formula (I) or its salt of the present invention has an excellent melanogenesis inhibiting effect and an antimicrobial effect, and is excellent in stability. Therefore, the melanogenesis inhibitor of the present invention prepared by formulating this propyl-phenyl-ether derivative as an active ingredient shows an excellent melanogenesis inhibiting effect and a skin-lightening effect, and is excellent in stability and used as a skin-lightening agent. Further, the antimicrobial agent of the present invention prepared by formulating this propyl-phenyl-ether derivative as an active ingredient has an excellent antimicrobial effect. Therefore, by formulating this propyl-phenyl-ether derivative, a cosmetic having an excellent melanogenesis inhibiting effect, a skin-lightening effect and an antimicrobial effect and being excellent in stability is obtained. Particularly, the cosmetic of the present invention is suitably used as a cosmetic having an excellent skin-lightening effect.

(Modes for Carrying Out the Invention)

Embodiments for carrying out the present invention will be explained below. The scope of the present invention is not limited to the embodiments.

Specific examples of the propyl-phenyl-ether derivative compound of the present invention include, for example, compounds shown below.

1-O-alkyl-2-hydroxy-3-(4'-alkylphenoxy)propyl
1-O-alkyl-2-hydroxy-3-(2'-alkylphenoxy)propyl
1-O-alkyl-2-hydroxy-3-(3'-alkylphenoxy)propyl
1-alkyl-2-hydroxy-3-(4'-alkylphenoxy)propyl
1-alkyl-2-hydroxy-3-(2'-alkylphenoxy)propyl
1-alkyl-2-hydroxy-3-(3'-alkylphenoxy)propyl
1,2-di-hydroxy-3-(4'-alkylphenoxy)propyl
1,2-di-hydroxy-3-(2'-alkylphenoxy)propyl
1,2-di-hydroxy-3-(3'-alkylphenoxy)propyl
1,3-di-hydroxy-3-(4'-alkylphenoxy)propyl
1,3-di-hydroxy-3-(2'-alkylphenoxy)propyl
1,3-di-hydroxy-3-(3'-alkylphenoxy)propyl
1-O-alkyl-3-hydroxy-2-(4'-alkylphenoxy)propyl
1-O-alkyl-3-hydroxy-2-(2'-alkylphenoxy)propyl
1-O-alkyl-3-hydroxy-2-(3'-alkylphenoxy)propyl
1-alkyl-3-hydroxy-2-(4'-alkylphenoxy)propyl
1-alkyl-3-hydroxy-2-(2'-alkylphenoxy)propyl
1-alkyl-3-hydroxy-2-(3'-alkylphenoxy)propyl
1-O-alkyl-2-hydroxy-3-(2',4'-di-alkylphenoxy)propyl

1-alkyl-2-hydroxy-3-(2',4'-alkylphenoxy)propyl
1,2-di-hydroxy-3-(2',4'-alkylphenoxy)propyl
1,3-di-hydroxy-2-(2',4'-alkylphenoxy)propyl
1-N-alkyl-2-hydroxy-3-(4'-alkylphenoxy)propyl
1-N-alkyl-2-hydroxy-3-(2'-alkylphenoxy)propyl
1-N-alkyl-2-hydroxy-3-(3'-alkylphenoxy)propyl
1-N-alkyl-3-hydroxy-2-(4'-alkylphenoxy)propyl
1-N-alkyl-3-hydroxy-2-(2'-alkylphenoxy)propyl
1-N-alkyl-3-hydroxy-2-(3'-alkylphenoxy)propyl
1-S-alkyl-2-hydroxy-3-(4'-alkylphenoxy)propyl
1-S-alkyl-2-hydroxy-3-(2'-alkylphenoxy)propyl
1-S-alkyl-2-hydroxy-3-(3'-alkylphenoxy)propyl
1-S-alkyl-3-hydroxy-2-(4'-alkylphenoxy)propyl
1-S-alkyl-3-hydroxy-2-(2'-alkylphenoxy)propyl
1-S-alkyl-3-hydroxy-2-(3'-alkylphenoxy)propyl
1-acyl-2-hydroxy-3-(4'-alkylphenoxy)propyl
1-acyl-2-hydroxy-3-(2'-alkylphenoxy)propyl
1-acyl-2-hydroxy-3-(3'-alkylphenoxy)propyl
1-phosphoryl-2-hydroxy-3-(4'-alkylphenoxy)propyl
1-phosphoryl-2-hydroxy-3-(2'-alkylphenoxy)propyl
1-phosphoryl-2-hydroxy-3-(3'-alkylphenoxy)propyl

In the above-described examples, O-alkyl denotes an alkoxy group, N-alkyl denotes an alkylamino group or a diaminoalkyl group, and S-alkyl denotes a thioalkoxy group.

The alkyl in the above-exemplified compounds includes linear alkyl groups such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, a behenyl group and the like,
branched alkyl groups such as an isopropyl group, an isobutyl group, a sec-butyl group, a tert-butyl group, an isopentyl group, a neopentyl group, a sec-pentyl group, a tert-pentyl group, an isohexyl group, a neohexyl group, a sec-hexyl group, a tert-hexyl group, a 2-methylpentyl group, a 3-methylpentyl group, a 2,2-dimethylbutyl group, a 2-ethylbutyl group, an isoheptyl group, an isooctyl group, a neooctyl group, a sec-octyl group, a tert-octyl group, an isononyl group, an isodecyl group, a neodecyl group, a sec-decyl group, a tert-decyl group, an isoundecyl group, an isododecyl group, an isotridecylglycidyl group, an isotetradecyl group, an isopentadecyl group, an isohexadecyl group, an isoheptadecyl group, an isohexadecyl group, an isooctadecyl group, an isononadecyl group, an isobehenyl group; and the like.

Specific examples of the propyl-phenyl-ether derivative of the present invention include also those prepared by substituting the alkyl in the above-exemplified compounds with an alkenyl group such as a vinyl group, an allyl group, a butenyl group, an isobutenyl group, a crotyl group, an octenyl group, a decenyl group, a dodecenyl group and the like.

The acyl in the above-exemplified compounds includes an acetyl group, a formyl group, a propanoyl group, a butanoyl group, a pentanoyl group, a hexanoyl group, a heptanoyl group, an octanoyl group, a nonayl group, a decanoyl group, an undecanoyl group, a dodecanoyl group, a tetradecanoyl group, a hexadecanoyl group, an octadecanoyl group, an eicosanoyl group, a hexadecenoyl group, an octadecenoyl group, an oleyl group, an octadecatrienoyl group, an icosatetraenoyl group, an isooctanoyl group, an isopalmitoyl group, an isostearoyl group, a 2-propylpentanoyl group, a 2-butylhexanoyl group, a 2-pentylheptanoyl group and the like.

Further, compounds obtained by substituting a phosphoryl group in the above-exemplified compounds with an alkylphosphoryl group are also included in the present invention.

The propyl-phenyl-ether derivative of the present invention can be produced by already known various methods. Examples thereof include a method of reacting a phenol compound represented by the following structural formula (IV) and an alkyl halide represented by the following structural formula (V).

(Chemical formula 18)

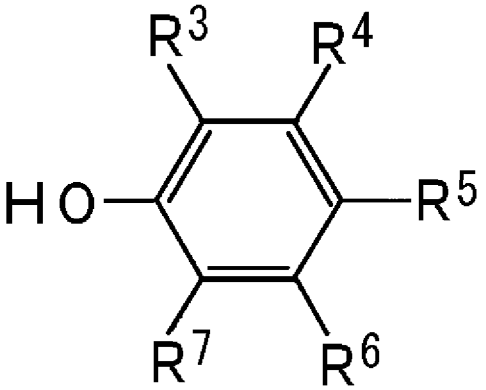

(IV)

R-X (V)

In the formula, $R^3$, $R^4$, $R^5$, $R^6$ and $R^7$ represent the same meaning as in the above-described general formula (I), and X represents a halogen.

Further, a method of reacting a phenol compound represented by the structural formula (IV) and an epoxy compound such as glycidol, glycidyl ether, epoxy alkane and the like, and a method of reacting a phenol compound represented by the structural formula (IV) and an alkylating agent such as an alkyl halide, a dialkyl sulfate and the like can be also mentioned. Further, an acylated body can be obtained by reacting the propyl-phenyl-ether derivative obtained in the above-described method with an acylating agent such as an acid anhydride, an acid halide and the like.

The kind of the solvent which can be used in the above-described reaction is not particularly restricted. The solvent includes, for example, water, lower alcohols such as methanol, ethanol, isopropanol and the like, dimethyl sulfoxide (DMSO), N,N-dimethylformamide (DMF), dioxane, tetrahydrofuran (THF), pyridine and the like, and mixed solvents thereof. The water-containing solvent includes one composed solely of water, and mixed solvents composed of water as the main component and a solvent selected from lower alcohols such as methanol, ethanol, isopropanol and the like, and DMSO, DMF, dioxane, THF, pyridine and the like. When the intended product can be obtained without using a solvent, a solvent may not be used.

The above-described reaction can also be carried out using a catalyst such as an acid catalyst, a basic catalyst, a phase transfer catalyst and the like.

The propyl-phenyl-ether derivative produced as described above can be purified by means such as column chromatography using silica gel, column chromatography using an ion exchange resin or the like, treatment with activated carbon, extraction, distillation, crystallization or the like.

In the cosmetic of the present invention, components usually used for cosmetic, for example, oily raw materials, surfactants, other moisturizing agents, polymers, antioxidants, other skin-lightening agents, ultraviolet absorbers, sequestrants, hydrolyzed protein, amino acids or derivative thereof, pH regulators, preservatives, thickeners, coloring matters, other medicines and the like can be appropriately formulated, in addition to the essential components.

Examples of the oily raw materials include oils and fats such as olive oil, camellia oil, macadamia nut oil, tea oil, castor oil and tri(caprone/capryl) glyceryl, waxes such as jojoba oil, carnauba wax, candelilla wax, lanolin and bees wax, hydrocarbons such as liquid paraffin, paraffin, vaseline, seresin, microcrystalline wax and squalane, fatty acids such as lauric acid, myristic acid, palmitic acid, stearic acid, behenic acid and isostearic acid, higher alcohols such as cetyl alcohol, stearyl alcohol and isostearyl alcohol, esters such as isopropyl myristate, 2-octyldodecyl myristate, cetyl 2-ethylhexanoate, diisostearyl malate and tri-2-ethylhexanoin, and silicones such as methyl polysiloxane, methyphenyl polysiloxane and decamethyl cyclopenta siloxane.

Examples of the surfactants include anionic surfactants such as higher fatty acid soaps, polyoxyethylene alkyl ether sulfate, acyl-N-methyl taurate, N-acyl amino acid salts and alkyl phosphates, cationic surfactants such as alkyl trimethyl ammonium chloride and dialkyl dimethyl ammonium chloride, ampholytic surfactants such as alkyl dimethyl aminoacetic acid betaine, alkyl amide aminoacetic acid betaine and 2-alkyl-N-carboxy-N-hydroxy imidazolynium betaine, and nonionic surfactants such as polyoxyethylene alkyl ether, polyethylene glycol fatty acid ester, polyhydric alcohol fatty acid ester and polyether-modified silicone.

Examples of the moisturizing agents include glycerin, propylene glycol, maltitol, sorbitol, 1,3-butylene glycol, sodium lactate, polyethylene glycol, sodium pyrrolidone carboxylate and sodium hyaluronate.

Examples of the polymers include carboxy vinyl polymer, carboxy methylcellulose sodium, xanthan gum, polyvinyl alcohol and dimethylpolysiloxane polymer. Examples of the antioxidants include vitamin E, tannin and BHT (butylhydroxytoluene).

**Though** the propyl-phenyl-ether derivative of the present invention has skin-lightening effect, other skin-lightening agents can be compounded into the cosmetic of the present invention. Examples of the other skin-lightening agents include ellagic acid, kojic acid, chamomile extract, liquorice extract, rucinol, rosemary extract, arbutin, tranexamic

acid, potassium 4-methoxysalicylate, ascorbic acid; ascorbic acid derivatives such as glyceryl ascorbic acid, ascorbic acid glucoside and magnesium ascorbyl phosphate; and the like.

Examples of the ultraviolet absorber includes ethylhexyl methoxycinnamate, octocrylene, 4-tert-butyl4'-methoxydibenzoylmethane, hexyl diethylaminohydroxybenzoylbenzoate and the like. Examples of the sequestering agent includes citramalic acid, agaric acid, glyceric acid, shikimic acid, Hinokitiol, gallic acid, tannic acid, caffeic acid, ethylenediaminetetraacetic acid, ethylene glycol diaminetetraacetic acid, diethylenetriaminepentaacetic acid, phytic acid, polyphosphoric acid, metaphosphoric acid, and analogs thereof, and alkali metal salts and carboxylates of them, and the like.

Examples of the hydrolyzed protein include protein hydrolysates such as milk protein, silk protein, wheat protein, rice protein, pea protein, collagen, keratin, soybean, sesame, conchiolin and marine collagen; derivatives thereof, and the like. Examples of the amino acid or its derivative include amino acids such as glycine, valine, leucine, isoleucine, serine, threonine, phenylalanine, arginine, lysine, asparagine, aspartic acid, glutamine, glutaminic acid, cystine, cysteine, methionine, tryptophan, proline, histidine and the like, and derivatives thereof.

Examples of the pH regulator include lactic acid, citric acid, glycolic acid, succinic acid, tartaric acid, malic acid, potassium carbonate, sodium hydrogen carbonate, ammonium hydrogen carbonate and the like. Examples of the preservative include alkyl p-oxybenzoates, benzoic acid, sodium benzoate, sorbic acid, potassium sorbate, phenoxyethanol and the like.

Examples of the thickener include gum Arabic, tragacanth gum, carob gum, guar gum, pectin, agar, quince seed, starch, algae colloid, xanthan gum, dextran, succinoglucan, collagen, gelatin, casein, albumin, carboxymethyl starch, methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, sodium alginate, polyvinyl methyl ether, carboxy vinyl polymer, sodium polyacrylate, polyethylene acrylate, polyacrylamide, cation polymer and the like.

Examples of the coloring matter include tar dye, natural colorants, inorganic pigments, polymer powders and the like. Examples of the perfume include natural perfumes, synthetic perfumes, blended perfumes and the like.

The other agents include rough skin preventing agents and anti-inflammatory agents. Examples of the rough skin preventing agent and anti-inflammatory agent include dipotassium glycyrrhizinate, steary glycirrhetinate, methyl salicylate, pyridoxine hydrochloride, allantoin, marine salt, mulberry root extract, aloe extract, gardenia florida extract, chamomile extract, liquorice extract, soapberry peel extract, apricot kernel extract, scutellaria root extract, sweet tea extract, loquat extract, ginkgo biloba extract, hypericum extract, yarrow extract, safflower extract, bitter orange peel extract, sage leaf extract, birch extract, citrus unshiu peel extract, peach kernel extract, mugwort extract, althea extract, arnica extract, ginseng extract, paeony root extract, cnidium officinale root extract, gentian extract, cordyceps sinensis extract, phellodendron bark extract, artemisia capillaris flower extract, geranium thunbergi extract, peach leaf extract, sasa albo-marginata extract, job's tears extract, horse chestnut extract, crataegus cuneata fruit extract, coptis japonica root extract, mushroom extract, calendula officinalis extract, peppermint extract, symphytum officinale extract, butcher's broom extract, malva sylvestris flower extract, rodgersia podophylla extract, rosa roxburghii fruit extract and the like. Additionally, an agent for hair growth, an agent for acne, an agent for dandruff and itching, an underarm odor-preventing agent and the like are also listed as the other agents.

The form of the cosmetic of the present invention is arbitrary. Any of a solution system, solubilization system, emulsion system, gel system, powder dispersion system, water-oil two-layer system and the like are possible. According to the intended cosmetic product, a hydroxypropylalkylphenyl ether derivative represented by the above-described general formula (I) or its salt and the above-described optional compounding components can be compounded.

Next, specific embodiments for carrying out the present invention will be explained concretely by examples. The scope of the present invention is not limited to the examples.

Synthesis Example 1

Synthesis of 1-(4'-tert-butylphenoxy)propyl

To 4-tert-butylphenol (1.00 g) were added sodium hydroxide (0.26 g) and tetrabutylammonium bromide (0.43 g) and the mixture was stirred at room temperature. Then, propyl bromide (1.64 g) was added and the mixture was further stirred at 50°C for 5 hours. Thereafter, ethyl acetate and water were added and the intended product was extracted with ethyl acetate. The extraction solution was dried over anhydrous sodium sulfate, then, concentrated under reduce pressure to obtain 1.37 g of the residue which was then subjected to silica gel column chromatography. It was eluted with a mixed liquid of hexane/ethyl acetate = 20/1, and concentrated under reduced pressure, to obtain the product. For the resultant product, a spot showing different Rf value and UV absorption different from each raw material was recognized by HPTLC analysis. It can be considered from the raw materials that the resultant product is 1-(4'-tert-butylphenoxy)propyl represented by the following structural formula.

(Chemical formula 19)

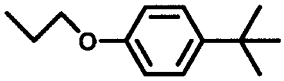

Synthesis Example 2

Synthesis of 1-(4'-tert-butylphenoxy)ethyl

To 4-tert-butylphenol (1.00 g) were added sodium hydroxide (0.26 g) and tetrabutylammonium bromide (0.43 g) and the mixture was stirred at room temperature. Then, ethyl bromide (2.20 g) was added and the mixture was further stirred at 40°C for 5 hours. Thereafter, ethyl acetate and water were added and the intended product was extracted with ethyl acetate. The extraction solution was dried over anhydrous sodium sulfate, then, concentrated under reduce pressure to obtain 0.97 g of the residue which was then subjected to silica gel column chromatography. It was eluted with a mixed liquid of hexane/ethyl acetate = 20/1, and concentrated under reduced pressure, to obtain the product. It was recognized by HPTLC analysis that the resultant product is a product having UV adsorption. It can be considered from the raw materials that the resultant product is 1-(4'-tert-butylphenoxy)ethyl represented by the following structural formula.

(Chemical formula 20)

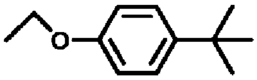

Synthesis Example 3

Synthesis of 1,2-di-hydroxy-3-phenoxypropyl

To phenylglycidyl ether (1.00 g) were added water (5.00 g) and DMSO(10.0 g) and the mixture was stirred. Then, concentrated sulfuric acid (0.06 g) was added and the mixture was further stirred at 80°C for 5 hours. Thereafter, ethyl acetate and water were added and the intended product was extracted with ethyl acetate. The extraction solution was dried over anhydrous sodium sulfate, then, concentrated under reduce pressure to obtain 0.85 g of the residue which was then subjected to silica gel column chromatography. It was eluted with a mixed liquid of hexane/ethyl acetate = 2/1, and concentrated under reduced pressure, to obtain the product. It was recognized by HPTLC analysis that the resultant product is a product having UV adsorption. It can be considered from the raw materials that the resultant product is 1,2-di-hydroxy-3-phenoxypropyl represented by the following structural formula.

(Chemical formula 21)

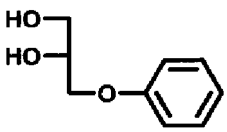

Synthesis Example 4

Synthesis of 1,2-di-hydroxy-3-(2'-methylphenoxy)propyl

To 2-metylphenol (1.00 g) were added sodium hydroxide (0.37 g) and tetrabutylammonium bromide (0.30 g) and the mixture was stirred at room temperature. Then, glycidol (0.75 g) was added and the mixture was further stirred at 50°C for 5 hours. Thereafter, ethyl acetate and water were added to conduct extraction. The extraction solution was dried over anhydrous sodium sulfate, then, concentrated under reduce pressure to obtain 1.49 g of the residue which was then subjected to silica gel column chromatography. It was eluted with a mixed liquid of hexane/ethyl acetate = 1/1,

and concentrated under reduced pressure, to obtain the product(0.47 g). It was recognized by HPTLC analysis that the resultant product is a product having UV adsorption. It can be considered from the raw materials that the resultant product is 1,2-di-hydroxy-3-(2'-methylphenoxy)propyl represented by the following structural formula.

(Chemical formula 22)

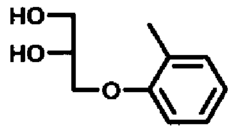

Example 1 Synthesis of 1,2-di-hydroxy-3-(4'-tert-butylphenoxy)propyl

To 4-tert-butylphenol (1.00 g) were added sodium hydroxide (0.26 g) and tetrabutylammonium bromide (0.43 g) and the mixture was stirred at room temperature. Then, glycidol (0.50 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.37 g of the residue which was then subjected to silica gel column chromatography. It was eluted with a mixed liquid of hexane/ethyl acetate =1/1, and concentrated under reduced pressure, to obtain the product (0.70 g).

For the resultant product, $^{1}$H-NMR measurement and $^{13}$C-NMR measurement were conducted. It was confirmed from the measured results that this product was 1,2-di-hydroxy-3-(4'-tert-butylphenoxy)propyl represented by the above-described structural formula (III).

The analyzed results by NMR were as described below. $^{1}$H-NMR (400MHz, $CDCl_3$) : $\delta$ ppm 1.29(9H,s), 2.49(OH,brs), 2.99(OH,brs), 3.74(1H,dd), 3.83(1H,dd), 4.02(2H,m), 4.10(1H,m), 6.85(2H,d), 7.30(2H,d)

$^{13}$C-NMR (100MHz, $CDCl_3$) : $\delta$ ppm 31.4, 34.1, 63.7, 69.1, 70.4, 113.9, 126.3, 144.0, 156.1

Example 2

Synthesis of 2-hydroxy-3-(4'-tert-butylphenoxy)propyl

To 4-tert-butylphenol (1.00 g) were added sodium hydroxide (0.53 g) and tetrabutylammonium bromide (0.43 g) and the mixture was stirred at room temperature. Then, propylene oxide (1.16 g) was added, and the mixture was further stirred at 50 °C for 5 hours. Thereafter, ethyl acetate and water were added and the intended product was extracted with ethyl acetate. The extraction solution was dried over anhydrous sodium sulfate, then, concentrated under reduced pressure to obtain 1.37 g of the residue which was then subjected to silica gel column chromatography. It was eluted with a mixed liquid of hexane/ethyl acetate =1/1, and concentrated under reduced pressure, to obtain the product (0.20 g).

For the resultant product, $^{1}$H-NMR measurement and $^{13}$C-NMR measurement were conducted. It was confirmed from the measured results that this product was 2-hydroxy-3-(4'-tert-butylphenoxy)propyl represented by the following structural formula.

(Chemical formula 23)

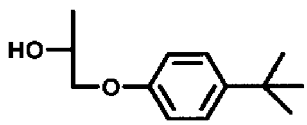

The analyzed results by NMR were as described below.

$^{1}$H-NMR (400MHz, $CDCl_3$) : $\delta$ ppm 1.29(9H,s), 1.35(3H,d), 1.62(OH,brs), 3.93(2H,dd), 5.24(1H,m), 6.85(2H,d), 7.30(2H,d)

$^{13}$C-NMR (100MHz, $CDCl_3$) : $\delta$ ppm 16.7, 21.3, 31.5, 34.1, 68.9, 70.0, 114.1, 126.2, 143.8, 156.3, 170.6

Example 3

Synthesis of 1-O-ethyl-2-hydroxy-3-(4'-tert-butylphenoxy)propyl and 2-O-ethyl-1-hydroxy-3-(4'-tert-butylphenoxy)propyl

To 4-tert-butylphenol (1.00 g) were added sodium hydroxide (0.26 g) and tetrabutylammonium bromide (0.43 g) and the mixture was stirred at room temperature. Then, ethyl glycidyl ether (0.50 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.37 g of the residue which was then subjected to silica gel column chromatography. It was eluted with a mixed liquid of hexane/ethyl acetate =1/1, and concentrated under reduced pressure, to obtain the product L (0.35 g) and product M (0.03 g).

For the product L, $^1$H-NMR measurement and $^{13}$C-NMR measurement were conducted. It was confirmed from the measured results that this product was 1-0-ethyl-2-hydroxy-3-(4'-tert-butylphenoxy)propyl represented by the above-described structural formula (II).

The analyzed results by NMR were as described below.

$^1$H-NMR (400MHz, $CDCl_3$) : $\delta$ ppm 1.22(3H,t), 1.30(9H,s), 3.59(4H,m), 4.01(2H,m), 4.15(1H,m), 6.86(2H,d), 7.30(2H,d)

$^{13}$C-NMR (100MHz, $CDCl_3$) : $\delta$ ppm 15.1, 31.5, 34.1, 66.9, 68.9, 69.1, 71.3, 114.0, 126.3, 143.8, 156.3

For the product M, $^1$H-NMR measurement and $^{13}$C-NMR measurement were conducted also. It was confirmed from the measured results that this product was 2-O-ethyl-1-hydroxy-3-(4'-tert-butylphenoxy)propyl represented by the following structural formula (VI).

The analyzed results by NMR were as described below.

$^1$H-NMR (400MHz, $CDCl_3$) : $\delta$ ppm 1.25(3H,t), 1.30(9H,s), 3.71(5H,m), 4.03(2H,t-like), 6.85(2H,d), 7.30(2H,d)

$^{13}$C-NMR (100MHz, $CDCl_3$) : $\delta$ ppm 15.7, 31.6, 34.2, 62.7, 66.0, 67.4, 78.0, 114.0, 126.3, 143.8, 156.4

(Chemical formula 24)

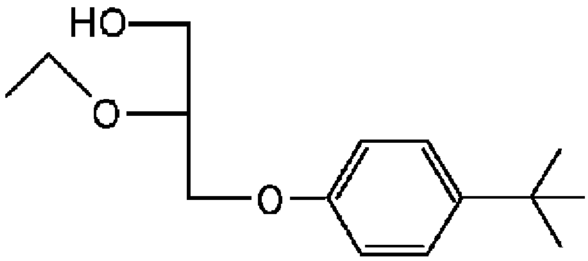

(VI)

Example 4

Synthesis of 1-O-ethyl-2-hydroxy-3-(4'-sec-butylphenoxy)propyl

To 4-sec-butylphenol (1.00 g) were added sodium hydroxide (0.26 g) and tetrabutylammonium bromide (0.43 g) and the mixture was stirred at room temperature. Then, ethyl glycidyl ether (0.50 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.17 g of the residue which was then subjected to silica gel column chromatography. It was eluted with a mixed liquid of hexane/ethyl acetate =1/1, and concentrated under reduced pressure, to obtain the product A (0.38 g) and product B (0.04 g).

For the product A, $^1$H-NMR measurement and $^{13}$C-NMR measurement were conducted. It was confirmed from the measured results that this product was 1-0-ethyl-2-hydroxy-3-(4'-sec-butylphenoxy)propyl represented by the above-described structural formula (A).

The analyzed results by NMR were as described below.

$^1$H-NMR (400MHz, $CDCl_3$) : $\delta$ ppm 0.80(3H,t), 1.21(6H,m), 1.55(2H,m), 2.54(1H,m), 3.59 (4H,m), 4.01(2H,m), 4.13(1H,m), 6.84(2H,d), 7.09(2H,d)

$^{13}$C-NMR (100MHz, $CDCl_3$) : $\delta$ ppm 12.2, 15.1, 22.0, 31.3, 40.8, 66.9, 68.9, 69.2, 70.5, 114.3, 128.0, 140.5, 156.4

It was recognized by HPTLC analysis that the product B is a product having UV adsorption. It can be considered from the raw materials and the reactivity that the product B is 1-hydroxy-2-O-ethyl-3-(4'-sec-butylphenoxy)propyl represented by the following structural formula(B).

(Chemical formula 25)

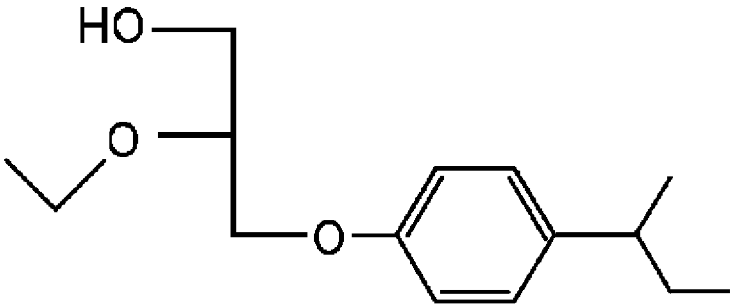

(B)

Example 5

Synthesis of 1,2-di-hydroxy-3-(4'-sec-butylphenoxy)propyl

To 4-sec-butylphenol (1.00 g) were added sodium hydroxide (0.26 g) and tetrabutylammonium bromide (0.43 g) and the mixture was stirred at room temperature. Then, glycidol (0.50 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.26 g of the residue which was then subjected to silica gel column chromatography. It was eluted with a mixed liquid of hexane/ethyl acetate =1/1, and concentrated under reduced pressure, to obtain the resultant product (0.65 g).

For the resultant product, $^1$H-NMR measurement and $^{13}$C-NMR measurement were conducted. It was confirmed from the measured results that this product was 1,2-di-hydroxy-3-(4'-sec-butylphenoxy)propyl represented by the above-described structural formula (D).

The analyzed results by NMR were as described below.

$^1$H-NMR (400MHz, $CDCl_3$) : $\delta$ ppm 0.80 (3H,t), 1.20 (3H,d), 1.55(2H,m), 2.54(1H,m), 3.74(1H,dd), 3.83(1H,dd), 4.01(1H,m), 4.10(1H,m), 6.84(2H,d), 7.09(2H,d)

$^{13}$C-NMR (100MHz, $CDCl_3$) : $\delta$ ppm 12.2, 22.0, 31.3, 40.8, 63.7, 69.2, 70.5, 114.3, 128.0, 140.5, 156.4

Example 6

Synthesis of 1,2-di-hydroxy-3-(2'-sec-butylphenoxy) propyl

To 2-sec-butylphenol (1.00 g) were added sodium hydroxide (0.26 g) and tetrabutylammonium bromide (0.43 g) and the mixture was stirred at room temperature. Then, glycidol (0.50 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.43 g of the residue which was then subjected to silica gel column chromatography. It was eluted with a mixed liquid of hexane/ethyl acetate =1/1, and concentrated under reduced pressure, to obtain the product (0.65 g).

For the resultant product, $^1$H-NMR measurement and $^{13}$C-NMR measurement were conducted. It was confirmed from the measured results that this product was 1,2-di-hydroxy-3-(2'-sec-butylphenoxy)propyl represented by the following structural formula (P).

(Chemical formula 26)

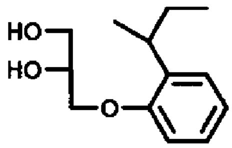

(P)

The analyzed results by NMR were as described below.
$^1$H-NMR (400MHz, $CDCl_3$) : δ ppm 0.84(3H,t), 1.20(3H,d), 1.60(2H,m), 2.66(OH,brs), 3.06(1H,m), 3.78(1H,ddd), 3.87(1H,dd), 4.05(2H,d), 4.14(1H,m), 6.85(1H,d), 6.96(2H,dt-like), 7.16(2H,m)
$^{13}$C-NMR (100MHz, $CDCl_3$) : δ ppm 12.2, 22.5, 29.9, 33.6, 63.9, 69.3, 70.6, 114.5, 121.3, 126.6, 126.9, 135.9, 155.7

Example 7

Synthesis of 1,2-di-octanoyl-3-(4'-tert-butylphenoxy) propyl

To 1,2-di-hydroxy-3-(4'-tert-butylphenoxy)propyl (1.00 g) obtained in Example 1 were added DMF (7 mL) and trimethylamine (0.30 g) and the mixture was stirred at room temperature, and then n-caprylic anhydride (0.80 g) was added. The mixture was stirred at 80°C for 2 hours, then, ethyl acetate and water were added and the intended product was extracted with ethyl acetate. The extraction solution was dried over anhydrous sodium sulfate, then, concentrated under reduced pressure to obtain 1.97 g of the residue which was then subjected to silica gel column chromatography. It was eluted with a mixed liquid of hexane/ethyl acetate = 5/1, and concentrated under reduced pressure, to obtain the product (0.65 g).
For the resultant product, $^1$H-NMR measurement and $^{13}$C-NMR measurement were conducted. It was confirmed from the measured results that this product was 1,2-di-octanoyl-3-(4'-tert-butylphenoxy) propyl represented by the above-described structural formula (C).
The analyzed results by NMR were as described below.
$^1$H-NMR (400MHz, $CDCl_3$) : δ ppm 0.88(6H,m), 1.30(25H,s), 1.63(4H,m), 2.36(4H,m), 4.01(2H,m), 4.26(3H,m), 6.85(2H,d), 7.31(2H,d)
$^{13}$C-NMR (100MHz, $CDCl_3$) : δ ppm 14.1, 22.6, 24.7, 24.9, 29.0, 29.07, 29.13, 34.09, 34.14, 34.20, 65.2, 68.7, 68.65, 68.70, 114.0, 126.4, 144.1, 156.1, 174.2, 179.8

Example 8

Synthesis of 2-hydroxy-1,3-di-(4'-tert-butylphenoxy) propyl

To epichlorohydrin (1.00 g) were added sodium hydroxide (0.43 g) and tetrabutylammonium bromide (0.43 g) and the mixture was stirred at room temperature. Then, 4-tert-butylphenol (1.96 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.87 g of the residue. To the residue, were added tetrabutylammonium bromide (0.19 g) and sodium hydroxide (0.24 g), and then 4-tert-butylphenol (1.96 g) was added. The mixture was stirred at 80°C for 2 hours, then, ethyl acetate and water were added and extraction was performed. The extraction solution was dried over anhydrous sodium sulfate, then, concentrated under reduced pressure to obtain 2.56 g of the residue which was then subjected to silica gel column chromatography. It was eluted with a mixed liquid of hexane/ethyl acetate = 10/1, and concentrated under reduced pressure, to obtain the resultant product (0.48 g).
For the resultant product, $^1$H-NMR measurement and $^{13}$C-NMR measurement were conducted. It was confirmed from the measured results that this product was 2-hydroxy-1,3-di-(4'-tert-butylphenoxy)propyl represented by the following structural formula.

(Chemical formula 27)

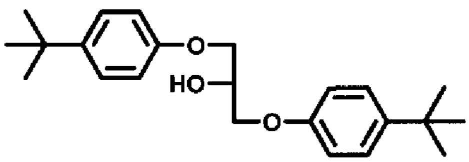

(K)

The analyzed results by NMR were as described below. $^{1}$H-NMR (400MHz, $CDCl_3$) : δ ppm 1.30(18H,s), 1.63(OH,brs), 2.64(OH,brs), 4.13(4H,m), 4.17(1H,m), 6.87(4H,m), 7.28(4H,d) $^{13}$C-NMR (100MHz, $CDCl_3$) : δ ppm 31.4, 34.1, 68.7, 68.8, 114.0, 126.3, 143.9, 156.1

Example 9

Synthesis of 1-(4" -hydroxyphenoxy)-2-hydroxy-3-(4'-tert-butylphenoxy)propyl

To epichlorohydrin (1.00 g) were added sodium hydroxide (0.43 g) and tetrabutylammonium bromide (0.43 g) and the mixture was stirred at room temperature. Then, 4-tert-butylphenol (1.96 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.87 g of the residue. To the residue, were added tetrabutylammonium bromide (0.19 g) and sodium hydroxide (0.24 g), and then hydroquinone (1.43 g) was added. The mixture was stirred at 80°C for 2 hours, then, ethyl acetate and water were added and extraction was performed. The extraction solution was dried over anhydrous sodium sulfate, then, concentrated under reduced pressure to obtain 2.56 g of the residue which was then subjected to silica gel column chromatography. It was eluted with a mixed liquid of hexane/ethyl acetate = 3/1, and concentrated under reduced pressure, to obtain the resultant product (0.27 g).

For the resultant product, $^{1}$H-NMR measurement and $^{13}$C-NMR measurement were conducted. It was confirmed from the measured results that this product was 1-(4" - hydroxyphenoxy)-2-hydroxy-3-(4'-tert-butylphenoxy)propyl represented by the following structural formula.

(Chemical formula 28)

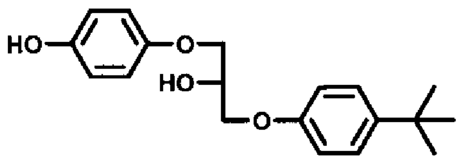

The analyzed results by NMR were as described below.
$^{1}$H-NMR (400MHz, $CDCl_3$) : δ ppm 1.30(9H,s), 1.64(OH,brs), 2.64(OH,brs), 4.10(4H,m), 4.13(1H,brs), 6.81(4H,m), 7.31 $^{13}$C-NMR (100MHz, $CDCl_3$) : δ ppm 31.5, 34.1, 68.7, 68.8, 69.4, 114.0, 115.5, 126.3, 144.0, 153.0, 156.1

Example 10

Synthesis of di[2-hydroxy-3-(4'-tert-butylphenoxy) propyl]ether

To epichlorohydrin (1.00 g) were added sodium hydroxide (0.43 g) and tetrabutylammonium bromide (0.43 g) and the mixture was stirred at room temperature. Then, 4-tert-butylphenol (1.96 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.87 g of the residue. To the residue, were added tetrabutylammonium bromide (0.19 g) and sodium hydroxide (0.24 g), and then 1,2-di-hydroxy-3-(4'-tert-butylphenoxy)propyl (2.19 g) obtained in Example 1 was added. The mixture was stirred at 80°C for 2 hours, then, ethyl acetate and water were added and extraction was performed. The extraction solution was dried over anhydrous sodium sulfate, then, concentrated under reduced pressure to obtain 2.56 g of the residue which was then subjected to silica gel column chromatography. It was eluted with a mixed liquid of hexane/ethyl acetate = 5/1, and concentrated under reduced pressure, to obtain the resultant product (0.35 g).

For the resultant product, $^{1}$H-NMR measurement and $^{13}$C-NMR measurement were conducted. It was confirmed from the measured results that this product was di[2-hydroxy-3- (4'-tert-butylphenoxy) propyl]ether represented by the

following structural formula.

(Chemical formula 29)

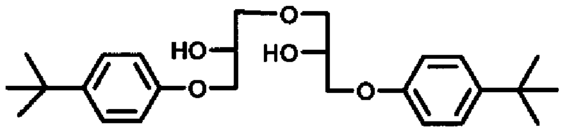

The analyzed results by NMR were as described below.
$^1$H-NMR (400MHz, $CDCl_3$) : $\delta$ ppm 1.29(18H,s), 1.67(OH,brs), 2.82(OH,brs), 3.28(OH,brs), 3.71(2H,m), 3.85(2H,m), 3.98(4H,m), 4.14(2H,m), 6.84(4H,m), 7.29(4H,m)
$^{13}$C-NMR (100MHz, $CDCl_3$) : $\delta$ ppm 31.5, 34.1, 67.7, 67.8, 68.5, 68.7, 69.08, 69.15, 69.18, 69.42, 71.5, 71.6, 72.1, 72.4, 72.7, 114.0, 126.24, 126.29, 143.7, 143.8, 143.9, 156.1, 156.2, 156.3

Example 11

Synthesis of 1-O-cetyl-2-hydroxy-3-(4'-tert-butylphenoxy)propyl

To epichlorohydrin (1.00 g) were added sodium hydroxide (0.43 g) and tetrabutylammonium bromide (0.43 g) and the mixture was stirred at room temperature. Then, 4-tert-butylphenol (1.96 g) was added, and the mixture was further stirred at room temperature for 5 hours. Thereafter, ethyl acetate and water were added and extraction was performed. The extraction solution was dried over anhydrous sodium sulfate, then, concentrated under reduced pressure to obtain 1.87 g of the residue. To the residue, were added cetyl alcohol (3.16 g) and concentrated sulfuric acid (0.21 g). The mixture was stirred at 80°C for 24 hours, then, ethyl acetate and water were added and the intended product was extracted with ethyl acetate. The extraction solution was dried over anhydrous sodium sulfate, then, concentrated under reduced pressure to obtain 3.02 g of the residue which was then subjected to silica gel column chromatography. It was eluted with a mixed liquid of hexane/ethyl acetate = 2/1, and concentrated under reduced pressure, to obtain the resultant product (0.30 g).
For the resultant product, $^1$H-NMR measurement and $^{13}$C-NMR measurement were conducted. It was confirmed from the measured results that this product was 1-0-cetyl-2-hydroxy-3-(4'-tert-butylphenoxy)propyl represented by the following structural formula.

(Chemical formula 30)

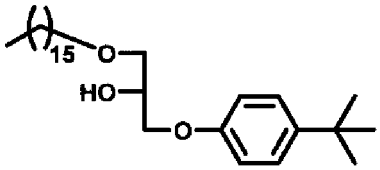

The analyzed results by NMR were as described below.
$^1$H-NMR (400MHz, $CDCl_3$) : $\delta$ ppm 0.86(9H,t), 1.24, 1.28(35H,s), 1.56(2H,m), 3.46(2H,m), 3.56(2H,m), 3.99(2H,m), 4.13(1H,m), 6.84(2H,d), 7.28(2H,d)
$^{13}$C-NMR (100MHz, $CDCl_3$) : $\delta$ ppm 14.1, 22.7, 26.1, 29.3, 29.5, 29.6, 29.7, 31.5, 31.9, 34.0, 68.9, 69.1, 71.4, 71.7, 114.0, 126.2, 143.7, 156.3

Example 12

Synthesis of 1,2-di-hydroxy-3-(4'-n-butylphenoxy)propyl

To 4-n-butylphenol (1.00 g) were added sodium hydroxide (0.26 g) and tetrabutylammonium bromide (0.43 g) and the mixture was stirred at room temperature. Then, glycidol (0.50 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure

to obtain 1.28 g of the residue which was then subjected to silica gel column chromatography. It was eluted with a mixed liquid of hexane/ethyl acetate =2/1, and concentrated under reduced pressure, to obtain the resultant product (0.58 g).

For the resultant product, $^1$H-NMR measurement and $^{13}$C-NMR measurement were conducted. It was confirmed from the measured results that this product was 1,2-di-hydroxy-3-(4'-n-butylphenoxy)propyl represented by the following structural formula.

(Chemical formula 31)

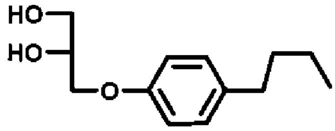

The analyzed results by NMR were as described below.
$^1$H-NMR (400MHz, $CDCl_3$) : $\delta$ ppm 0.90(3H,t), 1.32(2H,m), 1.54(2H,m), 2.52(2H,t), 3.71(1H,dd), 3.80(1H,dd), 3.98(2H,m), 4.07(1H,m), 6.80(2H,d), 7.06(2H,d)
$^{13}$C-NMR (100MHz, $CDCl_3$) : $\delta$ ppm 13.9, 22.2, 33.8, 34.7, 63.7, 69.1, 70.4, 114.3, 129.3, 135.7, 156.3

Example 13

Synthesis of 1,2-di-hydroxy-3-(4'-isopropylphenoxy) propyl

To 4-isopropylphenol (1.00 g) were added sodium hydroxide (0.29 g) and tetrabutylammonium bromide (0.24 g) and the mixture was stirred at room temperature. Then, glycidol (0.60 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.38 g of the residue which was then subjected to silica gel column chromatography. It was eluted with a mixed liquid of hexane/ethyl acetate =2/1, and concentrated under reduced pressure, to obtain the resultant product (0.53 g).

For the resultant product, $^1$H-NMR measurement and $^{13}$C-NMR measurement were conducted. It was confirmed from the measured results that this product was 1,2-di-hydroxy-3-(4'-isopropylphenoxy)propyl represented by the following structural formula.

(Chemical formula 32)

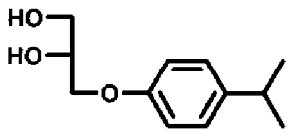

The analyzed results by NMR were as described below. $^1$H-NMR (400MHz, $CDCl_3$): $\delta$ ppm 1.20(6H,d), 2.84(1H,m), 3.72(1H,dd), 3.81(1H,dd), 3.99(2H,d-like), 4.08(1H,m), 6.82(2H,d), 7.12(2H,d)
$^{13}$C-NMR (100MHz, $CDCl_3$): $\delta$ ppm 24.1, 33.2, 63.7, 69.2, 70.4, 114.3, 127.3, 141.7, 156.4

Example 14

Synthesis of 1,2-di-hydroxy-3-(4'-ethylphenoxy)propyl

To 4-ethylphenol (1.00 g) were added sodium hydroxide (0.33 g) and tetrabutylammonium bromide (0.26 g) and the mixture was stirred at room temperature. Then, glycidol (0.67 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.26 g of the residue which was then subjected to silica gel column chromatography. It was eluted with a mixed liquid of hexane/ethyl acetate =2/1, and concentrated under reduced pressure, to obtain the resultant product (0.40 g).

For the resultant product, $^1$H-NMR measurement and $^{13}$C-NMR measurement were conducted. It was confirmed from the measured results that this product was 1,2-di-hydroxy-3-(4'-ethylphenoxy)propyl represented by the following structural formula.

(Chemical formula 33)

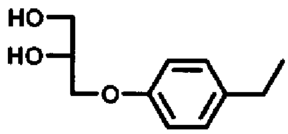

The analyzed results by NMR were as described below.
$^1$H-NMR (400MHz, $CDCl_3$): δ ppm 1.18(3H,s), 2.57(2H,d), 3.72(1H,dd), 3.81(1H,dd), 3.99(1H,brd), 4.07(1H,brd), 6.81(2H,d), 7.08(2H,d)
$^{13}$C-NMR (100MHz, $CDCl_3$): δ ppm 15.8, 27.9, 63.7, 69.2, 70.4, 114.4, 128.8, 137.1, 156.3

Example 15

Synthesis of 1,2-di-hydroxy-3-(4'-tert-amylphenoxy) propyl

To 4-tert-amylphenol (1.00 g) were added sodium hydroxide (0.24 g) and tetrabutylammonium bromide (0.20 g) and the mixture was stirred at room temperature. Then, glycidol (0.50 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.22 g of the residue which was then subjected to silica gel column chromatography. It was eluted with a mixed liquid of hexane/ethyl acetate =2/1, and concentrated under reduced pressure, to obtain the resultant product (0.50 g).

For the resultant product, $^1$H-NMR measurement and $^{13}$C-NMR measurement were conducted. It was confirmed from the measured results that this product was 1,2-di-hydroxy-3-(4'-tert-amylphenoxy)propyl represented by the following structural formula.

(Chemical formula 34)

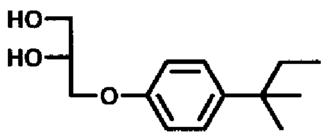

The analyzed results by NMR were as described below.
$^1$H-NMR (400MHz, $CDCl_3$) : δ ppm 0.65(3H,t), 1.35(9H,m), 1.59(1H, q), 3.58(4H,m), 4.00(2H,m), 4.15(1H,m), 6.84(2H,d), 7.22(2H, d)
$^{13}$C-NMR (100MHz, $CDCl_3$): δ ppm 9.1, 15.1, 28.6, 36.9, 37.3, 66.9, 68.9, 69.1, 71.3, 113.9, 126.9, 142.0, 156.2

Example 16

Synthesis of 1,2-di-hydroxy-3-(4'-α-cumylphenoxy)propyl

To 4-α-cumylphenol (1.00 g) were added sodium hydroxide (0.19 g) and tetrabutylammonium bromide (0.15 g) and the mixture was stirred at room temperature. Then, glycidol (0.38 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.05 g of the residue which was then subjected to silica gel column chromatography. It was eluted with a mixed liquid of hexane/ethyl acetate =2/1, and concentrated under reduced pressure, to obtain the resultant product (0.31 g).

For the resultant product, $^1$H-NMR measurement and $^{13}$C-NMR measurement were conducted. It was confirmed

from the measured results that this product was 1,2-di-hydroxy-3-(4'-α-cumylphenoxy)propyl represented by the following structural formula.

(Chemical formula 35)

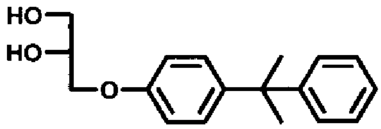

The analyzed results by NMR were as described below.
$^{1}$H-NMR (400MHz, $CDCl_3$): δ ppm 1.66(6H,s), (1H,m), 3.73(1H,dd), 3.83(1H,dd), 4.01(2H,m), 4.10(1H,m), 6.81(2H,d), 7.16(3H,m), 7.25(4H,m)
$^{13}$C-NMR (100MHz, $CDCl_3$) : δ ppm 30.8, 42.3, 63.7, 69.1, 70.4, 113.9, 125.6, 126.7, 127.86, 127.94, 143.6, 150.7, 156.2

Example 17

Synthesis of 1,2-di-hydroxy-3-[4'-(1", 1", 3", 3" - tetramethylbutyl)phenoxy]propyl

To 4-(1,1,3,3-tetramethylbuty)phenol (1.00 g) were added sodium hydroxide (0.19 g) and tetrabutylammonium bromide (0.15 g) and the mixture was stirred at room temperature. Then, glycidol (0.40 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.10 g of the residue which was then subjected to silica gel column chromatography. It was eluted with a mixed liquid of hexane/ethyl acetate =2/1, and concentrated under reduced pressure, to obtain the resultant product (0.40 g).

For the resultant product, $^{1}$H-NMR measurement and $^{13}$C-NMR measurement were conducted. It was confirmed from the measured results that this product was 1,2-di-hydroxy-3-[4'-(1" , 1", 3", 3" -tetramethylbutyl)phenoxy]propyl represented by the following structural formula (G).

(Chemical formula 36)

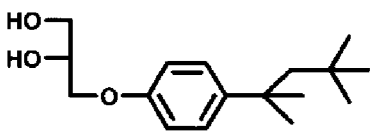

(G)

The analyzed results by NMR were as described below.
$^{1}$H-NMR (400MHz, $CDCl_3$): δ ppm 0.70(9H,s), 1.22(3H,m), 1.33(3H,s), 1.69(2H,s), 3.58(4H,m), 4.00(2H,m), 4.15(1H,m), 6.83(2H,d), 7.26(2H,d)
$^{13}$C-NMR (100MHz, $CDCl_3$): δ ppm 15.1, 31.6, 31.7, 32.3, 37.9, 56.9, 66.9, 68.9, 69.1, 71.3, 113.7, 127.1, 142.6, 156.2

Example 18

Synthesis of 1-hydroxy-3-(4'-tert-butylphenoxy)propyl

To 4-tert-butylphenol (1.00 g) were added sodium hydroxide (0.19 g) and tetrabutylammonium bromide (0.21 g) and the mixture was stirred at room temperature. Then, 3-bromo-1-hydroxypropyl (1.02 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.98 g of the residue which was then subjected to silica gel column chromatography. It was eluted with a mixed liquid of hexane/ethyl acetate =2/1, and concentrated under reduced pressure, to obtain the resultant product (0.38 g).

It was recognized by HPTLC analysis that the resultant product is a product having UV adsorption. It can be considered from the raw materials that the resultant product is 1-hydroxy-3-(4'-tert-butylphenoxy)propyl represented by

the following structural formula.

(Chemical formula 37)

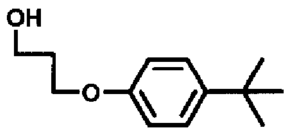

Example 19

Synthesis of 1,2-di-hydroxy-3-(2',6'-di-secbutylphenoxy)propyl

To 2,6-di-sec-butylphenol (1.00 g) were added sodium hydroxide (0.19 g) and tetrabutylammonium bromide (0.16 g) and the mixture was stirred at room temperature. Then, glycidol (0.40 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.24 g of the residue which was then subjected to silica gel column chromatography. It was eluted with a mixed liquid of hexane/ethyl acetate =3/1, and concentrated under reduced pressure, to obtain the resultant product (0.17 g).

It was recognized by HPTLC analysis that the resultant product is a product having UV adsorption. It can be considered from the raw materials that the resultant product is 1,2-di-hydroxy-3-(2',6'-di-sec-butylphenoxy)propyl represented by the following structural formula.

(Chemical formula 38)

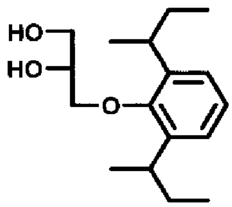

Example 20

Synthesis of 1,2-di-hydroxy-3-(2',4'-di-tert-butylphenoxy)propyl

To 2,4-di-tert-butyphenol (1.00 g) were added sodium hydroxide (0.19 g) and tetrabutylammonium bromide (0.16 g) and the mixture was stirred at room temperature. Then, glycidol (0.40 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.15 g of the residue which was then subjected to silica gel column chromatography. It was eluted with a mixed liquid of hexane/ethyl acetate =2/1, and concentrated under reduced pressure, to obtain the resultant product (0.31 g).

For the resultant product, $^{1}H$-NMR measurement and $^{13}C$-NMR measurement were conducted. It was confirmed from the measured results that this product was 1,2-di-hydroxy-3-(2',4'-di-tert-butylphenoxy)propyl represented by the following structural formula.

(Chemical formula 39)

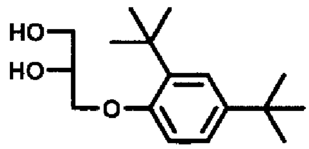

The analyzed results by NMR were as described below.
$^1$H-NMR (400MHz, $CDCl_3$): δ ppm 1.29(9H,s), 1.38(9H,s), 3.77(1H,dd), 3.88(1H,dd), 4.05(2H,m), 4.18(1H,m), 6.80(1H,d), 7.16(1H,m), 7.32(1H,d)
$^{13}$C-NMR (100MHz, $CDCl_3$): δ ppm 30.0, 31.5, 34.3, 35.0, 64.0, 68.9, 70.8, 111.5, 123.5, 124.1, 137.1, 143.2, 154.8

Example 21

Synthesis of 1,2-di-hydroxy-3-(2',4'-di-tert-butyl-5'-methylphenoxy)propyl

To 2,4-di-tert-buty-5-methylphenol (1.00 g) were added sodium hydroxide (0.18 g) and tetrabutylammonium bromide (0.15 g) and the mixture was stirred at room temperature. Then, glycidol (0.34 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.20 g of the residue which was then subjected to silica gel column chromatography. It was eluted with a mixed liquid of hexane/ethyl acetate =2/1, and concentrated under reduced pressure, to obtain the resultant product (0.27 g).

For the resultant product, $^1$H-NMR measurement and $^{13}$C-NMR measurement were conducted. It was confirmed from the measured results that this product was 1,2-di-hydroxy-3-(2',4'-di-tert-butyl-5'-methylphenoxy)propyl represented by the following structural formula.

(Chemical formula 40)

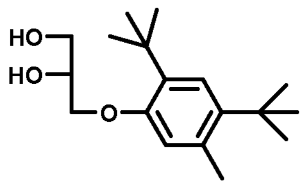

The analyzed results by NMR were as described below.
$^1$H-NMR (400MHz, $CDCl_3$): δ ppm 1.36(21H,brs), 3.76(1H,dd), 3.87(1H,dd), 4.06(2H,m), 4.16(1H,m), 6.63(1H,s), 7.29(1H,s)
$^{13}$C-NMR (100MHz, $CDCl_3$): δ ppm 22.8, 30.1, 31.1, 34.8, 35.5, 64.0, 68.9, 70.8, 116.5, 125.1, 134.2, 134.8, 140.0, 154.4

Example 22

Synthesis of 1,2-di-hydroxy-3-(2',6'-di-tert-butyl-4'-methylphenoxy)propyl

To 2,6-di-tert-butyl-4-methylphenol (1.00 g) were added sodium hydroxide (0.18 g) and tetrabutylammonium bromide (0.15 g) and the mixture was stirred at room temperature. Then, glycidol (0.34 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.15 g of the residue which was then subjected to silica gel column chromatography. It was eluted with a mixed liquid of hexane/ethyl acetate =2/1, and concentrated under reduced pressure, to obtain the resultant product (0.31 g).

It was recognized by HPTLC analysis that the resultant product is a product having UV adsorption. It can be considered from the raw materials that the resultant product is 1,2-di-hydroxy-3-(2',6'-di-tert-butyl-4'-methylphenoxy) propyl represented by the following structural formula.

(Chemical formula 41)

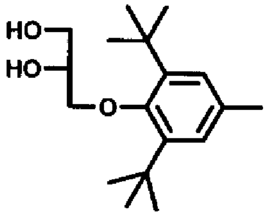

Example 23

Synthesis of 1,2-di-hydroxy-3-(2'-tert-butyl-4'-methoxylphenoxy)propyl

To 2-tert-butyl-4-methoxylphenol (1.00 g) were added sodium hydroxide (0.22 g) and tetrabutylammonium bromide (0.18 g) and the mixture was stirred at room temperature. Then, glycidol (0.45 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.39 g of the residue which was then subjected to silica gel column chromatography. It was eluted with a mixed liquid of hexane/ethyl acetate =2/1, and concentrated under reduced pressure, to obtain the resultant product (0.50 g).

It was recognized by HPTLC analysis that the resultant product is a product having UV adsorption. It can be considered from the raw materials that the resultant product is 1,2-di-hydroxy-3-(2'-tert-butyl-4'-methoxylphenoxy)propyl represented by the following structural formula.

(Chemical formula 42)

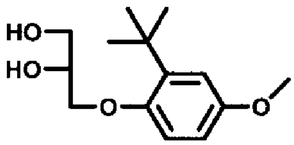

Example 24

Synthesis of 1,2-di-hydroxy-3-(3'-tert-butyl-4'-methoxylphenoxy)propyl

To 3-tert-butyl-4-methoxylphenol (1.00 g) were added sodium hydroxide (0.22 g) and tetrabutylammonium bromide (0.18 g) and the mixture was stirred at room temperature. Then, glycidol (0.45 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.20 g of the residue which was then subjected to silica gel column chromatography. It was eluted with a mixed liquid of hexane/ethyl acetate =2/1, and concentrated under reduced pressure, to obtain the resultant product (0.41 g).

It was recognized by HPTLC analysis that the resultant product is a product having UV adsorption. It can be considered from the raw materials that the resultant product is 1,2-di-hydroxy-3-(3'-tert-butyl-4'-methoxylphenoxy)propyl represented by the following structural formula.

(Chemical formula 43)

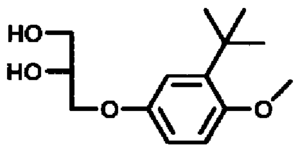

Example 25

Synthesis of 2,2-bis[4'-(1" ,2" -di-hydroxypropoxy) phenyl]propane

To 2,2-bis(4-glycidyloxypheny)propane (1.00 g) were added water (5.0 g) and DMSO (10.0 g) and the mixture was stirred at room temperature. Then, concentrated sulfuric acid (0.06 g) was added, and the mixture was further stirred at 80°C for 2 hours. Thereafter, ethyl acetate and water were added and the intended product was extracted with ethyl acetate. The extraction solution was dried over anhydrous sodium sulfate, then, concentrated under reduced pressure to obtain 1.54 g of the residue which was then subjected to silica gel column chromatography. It was eluted with a mixed liquid of hexane/ethyl acetate =3/1, and concentrated under reduced pressure, to obtain the resultant product (0.40 g).

It was recognized by HPTLC analysis that the resultant product is a product having UV adsorption. It can be considered from the raw materials that the resultant product is 2,2-bis[4'-(1" ,2" -di-hydroxypropoxy)phenyl]propane represented by the following structural formula.

(Chemical formula 44)

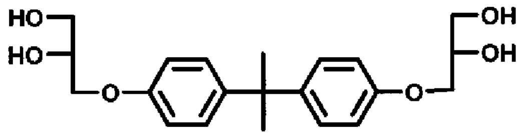

Example 26

Synthesis of 1-O-(2" -ethylhexyl)-2-hydroxy-3-(2'-sec-butylphenoxy)propyl

To 2-ethylhexylglycidylether (1.00 g) were added sodium hydroxide (0.22 g) and tetrabutylammonium bromide (0.17 g) and the mixture was stirred at room temperature. Then, 2-sec-butylphenol (0.89 g) was added, and the mixture was further stirred at room temperature for 5 hours. Thereafter, ethyl acetate and water were added and the intended product was extracted with ethyl acetate. The extraction solution was dried over anhydrous sodium sulfate, then, concentrated under reduced pressure to obtain 1.67 g of the residue which was then subjected to silica gel column chromatography. It was eluted with a mixed liquid of hexane/ethyl acetate =2/1, and concentrated under reduced pressure, to obtain the resultant product (0.10 g).

It was recognized by HPTLC analysis that the resultant product is a product having UV adsorption. It can be considered from the raw materials that the resultant product is 1-O-(2" -ethylhexyl)-2-hydroxy-3-(2'-sec-butylphenoxy) propyl represented by the following structural formula.

(Chemical formula 45)

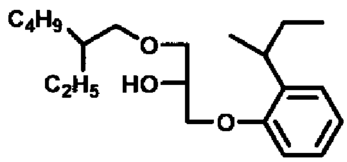

Example 27

Synthesis of 1-O-ethyl-2-hydroxy-3-(2'-sec-butylphenoxy) propyl

To 2-sec-butylphenol (1.00 g) were added sodium hydroxide (0.27 g) and tetrabutylammonium bromide (0.22 g) and the mixture was stirred at room temperature. Then, ethylglycidylether (0.75 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.69 g of the residue which was then subjected to silica gel column chromatography. It was eluted with a mixed liquid of hexane/ethyl acetate =2/1, and concentrated under reduced pressure, to obtain the resultant product (0.54 g).

For the resultant product, $^{1}$H-NMR measurement and $^{13}$C-NMR measurement were conducted. It was confirmed

from the measured results that this product was 1-0-ethyl-2-hydroxy-3-(2'-sec-butylphenoxy)propyl represented by the following structural formula (J).

(Chemical formula 46)

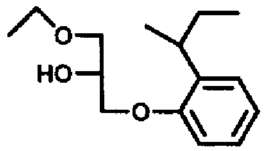

(J)

The analyzed results by NMR were as described below.
$^{1}$H-NMR (400MHz, $CDCl_3$) : $\delta$ ppm 0.83(3H,t), 1.23(6H,m), 1.57(2H,m), 3.07(1H,m), 3.59(4H,m), 4.01(2H,d-like), 4.17(1H,m), 6.84(1H,d), 6.93(1H,t-like), 7.14(2H,m)
$^{13}$C-NMR (100MHz, $CDCl_3$) : $\delta$ ppm 12.2, 15.1, 20.4, 29.9, 33.6, 66.9, 68.9, 69.2, 71.4, 111.5, 121.0, 126.5, 126.8, 135.9, 155.9

Example 28

Synthesis of 1-0-ethyl-2-hydroxy-3-(4'-$\alpha$-cumylphenoxy) propyl

To 4-cumylphenol (1.00 g) were added sodium hydroxide (0.19 g) and tetrabutylammonium bromide (0.15 g) and the mixture was stirred at room temperature. Then, ethylglycidylether (0.53 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.50 g of the residue which was then subjected to silica gel column chromatography. It was eluted with a mixed liquid of hexane/ethyl acetate =2/1, and concentrated under reduced pressure, to obtain the resultant product (0.47 g).

For the resultant product, $^{1}$H-NMR measurement and $^{13}$C-NMR measurement were conducted. It was confirmed from the measured results that this product was 1-0-ethyl-2-hydroxy-3-(4'-$\alpha$-cumylphenoxy)propyl 1 represented by the following structural formula.

(Chemical formula 47)

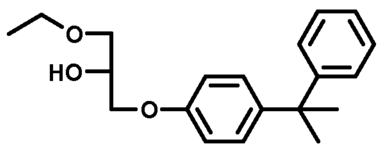

The analyzed results by NMR were as described below.
$^{1}$H-NMR (400MHz, $CDCl_3$): $\delta$ ppm 1.22(3H,t), 1.66(6H,m), 3.58(4H,m), 4.00(2H,m), 4.15(1H,m), 6.84(2H,d), 7.16(3H,m), 7.25(4H,m)
$^{13}$C-NMR (100MHz, $CDCl_3$): $\delta$ ppm 15.1, 30.8, 42.3, 66.9, 68.9, 69.1, 71.3, 113.9, 125.5, 126.7, 127.8, 127.9, 143.3, 150.8, 156.4

Example 29

Synthesis of 1-0-ethyl-2-hydroxy-3-(3'-methyl-4'-isoproylphenoxy)propyl

To 3-methyl-4-isoproylphenol (1.00 g) were added sodium hydroxide (0.27 g) and tetrabutylammonium bromide (0.22 g) and the mixture was stirred at room temperature. Then, ethylglycidylether (0.75 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.60 g of the residue which was then subjected to silica gel column chromatography. It was

eluted with a mixed liquid of hexane/ethyl acetate =2/1, and concentrated under reduced pressure, to obtain the resultant product (0.74 g).

For the resultant product, $^1$H-NMR measurement and $^{13}$C-NMR measurement were conducted. It was confirmed from the measured results that this product was 1-0-ethyl-2-hydroxy-3-(3'-methyl-4'-isoproylphenoxy)propyl represented by the following structural formula (F).

(Chemical formula 48)

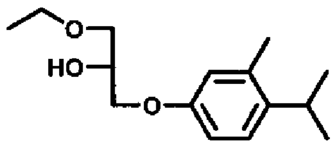

(F)

The analyzed results by NMR were as described below.

$^1$H-NMR (400MHz, $CDCl_3$): δ ppm 1.22(9H,t), 2.92(3H,s), 3.05(1H,m), 3.57(4H,m), 3.98(2H,m), 4.13(1H,m), 6.72(2H,d), 7.13(1H,d)

$^{13}$C-NMR (100MHz, $CDCl_3$): δ ppm 15.1, 19.4, 23.4, 28.6, 66.9, 68.8, 69.1, 71.3, 111.8, 116.4, 125.6, 136.4, 139.5, 156.2

Example 30

Synthesis of 1-O-ethyl-2-hydroxy-3-(2',6'-di-sec-butylphenoxy)propyl

To 2,6-di-sec-butylphenol (1.00 g) were added sodium hydroxide (0.19 g) and tetrabutylammonium bromide (0.16 g) and the mixture was stirred at room temperature. Then, ethylglycidylether (0.54 g) was added, and the mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.31 g of the residue which was then subjected to silica gel column chromatography. It was eluted with a mixed liquid of hexane/ethyl acetate =2/1, and concentrated under reduced pressure, to obtain the resultant product (0.39 g).

For the resultant product, $^1$H-NMR measurement and $^{13}$C-NMR measurement were conducted. It was confirmed from the measured results that this product was 1-0-ethyl-2-hydroxy-3-(2',6'-di-sec-butylphenoxy)propyl represented by the following structural formula.

(Chemical formula 49)

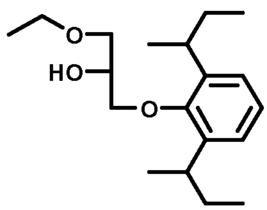

The analyzed results by NMR were as described below.

$^1$H-NMR (400MHz, $CDCl_3$): δ ppm 0.82(6H,m), 1.23(9H,s), 1.59(4H,s), 2.51(1H,m), 3.03(1H,m), 3.56(3H,m), 3.64(1H,dd), 3.99(2H,d-like), 4.16(1H,m), 6.77(1H,d), 6.93(2H,d)

$^{13}$C-NMR (100MHz, $CDCl_3$): δ ppm 12.2, 15.1, 20.4, 21.9, 22.0, 29.8, 31.3, 31.4, 33.8, 41.0, 66.9, 69.0, 69.3, 71.5, 111.3, 111.29, 111.32, 124.4, 124.5, 125.6, 125.8, 135.4, 140.1, 153.9

Example 31

Synthesis of 1,2-di-hydroxy-3-(3'-methyl-4'-isoproyl phenoxy)propyl

To 3-methyl-4-isoproylphenol (1.00 g) were added sodium hydroxide (0.27 g) and tetrabutylammonium bromide (0.22 g) and the mixture was stirred at room temperature. Then, glycidol (0.75 g) was added, and the mixture was further

stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.54 g of the residue which was then subjected to silica gel column chromatography. It was eluted with a mixed liquid of hexane/ethyl acetate =1/1, and concentrated under reduced pressure, to obtain the resultant product (0.68 g).

For the resultant product, $^1$H-NMR measurement and $^{13}$C-NMR measurement were conducted. It was confirmed from the measured results that this product was 1,2-di-hydroxy-3-(3'-methyl-4'-isoproylphenoxy)propyl represented by the following structural formula (E).

(Chemical formula 50)

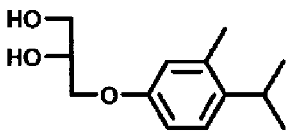

(E)

The analyzed results by NMR were as described below.
$^1$H-NMR (400MHz, $CDCl_3$): δ ppm 1.18(6H,d), 2.29(3H,s), 3.05(1H,m), 3.72(1H,dd), 3.81(1H,dd), 3.99(2H,m), 4.09(1H,m), 6.71(1H,2m), 7.14(1H,d)
$^{13}$C-NMR (100MHz, $CDCl_3$): δ ppm 19.4, 23.4, 63.7, 69.1, 70.4, 111.8, 116.3, 125.7, 136.5, 139.8, 155.9

Example 32

Synthesis of 2,2-bis[4'-(1" -O-ethyl-2" -hydroxypropoxy) phenyl]propane

To 2,2-bis(4-glycidyloxypheny)propane (1.00 g) were added ethanol (0.14 g) and concentrated sulfuric acid (0.03 g) and the mixture was stirred at 50°C for 5 hours. Then, ethyl acetate and water were added and the intended product was extracted with ethyl acetate. The extraction solution was dried over anhydrous sodium sulfate, then, concentrated under reduced pressure to obtain 1.04 g of the residue which was then subjected to silica gel column chromatography. It was eluted with a mixed liquid of hexane/ethyl acetate =1/1, and concentrated under reduced pressure, to obtain the resultant product (0.29 g).

For the resultant product, $^1$H-NMR measurement and $^{13}$C-NMR measurement were conducted. It was confirmed from the measured results that this product was 2,2-bis[4'-(1"-O-ethyl-2" -hydroxypropoxy)phenyl]propane represented by the following structural formula.

(Chemical formula 51)

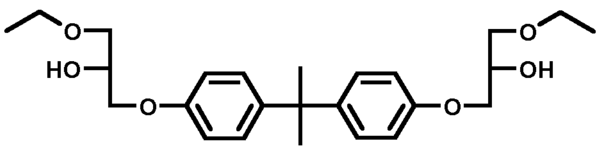

The analyzed results by NMR were as described below.
$^1$H-NMR (400MHz, $CDCl_3$) : δ ppm 1.19(6H,t), 1.61(6H,s), 2.50(4H,s), 3.56(8H,m), 3.99(4H,m), 4.13(2H,m), 6.80(2H,d), 7.11(2H,d)
$^{13}$C-NMR (100MHz, $CDCl_3$): δ ppm 15.1, 31.0, 41.7, 68.9, 69.0, 71.3, 113.9, 127.7, 143.5, 156.3

Example 33

Synthesis of 1-O-ethyl-2-hydroxy-3-[4'-(1", 1", 3" , 3" - tetramethylbutyl)phenoxy]propyl

To 4-(1,1,3,3-tetramethylbuty)phenol (1.00 g) were added sodium hydroxide (0.19 g) and tetrabutylammonium bromide (0.15 g) and the mixture was stirred at room temperature. Then, ethylglycidylether (0.55 g) was added, and the

mixture was further stirred at 50°C for 5 hours. Thereafter, ethyl acetate and water were added and the intended product was extracted with ethyl acetate. The extraction solution was dried over anhydrous sodium sulfate, then, concentrated under reduced pressure to obtain 1.23 g of the residue which was then subjected to silica gel column chromatography. It was eluted with a mixed liquid of hexane/ethyl acetate =2/1, and concentrated under reduced pressure, to obtain the resultant product (0.31 g).

For the resultant product, $^{1}$H-NMR measurement and $^{13}$C-NMR measurement were conducted. It was confirmed from the measured results that this product was 1-0-ethyl-2-hydroxy-3-[4'-(1", 1", 3", 3"-tetramethylbutyl)phenoxy]propyl represented by the following structural formula.

(Chemical formula 52)

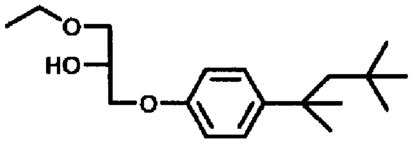

(H)

The analyzed results by NMR were as described below.
$^{1}$H-NMR (400MHz, $CDCl_3$): δ ppm 0.70(9H,s), 1.22(3H,m), 1.33(3H,s), 3.58(4H,m), 4.00(2H,m), 4.15(1H,m), 6.83(2H,d), 7.26(2H,d)
$^{13}$C-NMR (100MHz, $CDCl_3$): δ ppm 15.1, 31.6, 31.7, 32.3, 37.9, 56.9, 66.9, 68.9, 69.1, 71.3, 113.7, 127.1, 142.6, 156.2

Test Example 1 Melanogenesis inhibiting test

As a test for a skin-lightening effect, evaluation of the action of B16 melanoma 4A5 cell on theophylline-induced melanine production was carried out on propyl-phenyl-ether derivatives of the present invention, according to the following procedure. The results are shown in Tables 1 to 3.

(1) B16 mouse melanoma 4A5 strain was sowed onto a 48-well plate at a cell density of 8.0 x $10^3$ cells/well.
(2) Culturing was performed for 24 hours with Dulbecco's Modified Eagle's medium (manufactured by SIGMA. Hereinafter, abbreviated as D-MEM) containing 10% fetal bovine serum (manufactured by Nichirei Biosciences Inc.). Then, a 10% fetal bovine serum-containing D-MEM which contains theophylline so as to give the final concentration of 1.0 mmol/L and a 10% fetal bovine serum-containing D-MEM which contains a sample so as to give the final concentration of a predetermined concentration were added thereto.
(3) After culturing for 3 days in the co-existence of a sample, the medium was removed using an aspirator. Then, after distilled water was added, cells were broken by an ultrasonic wave.
(4) Thereafter, the amount of protein was determined using BCA protein assay kit (manufactured by Thermo Fisher Scientific Inc.), and the produced amount of melanine was measured by an alkali solubilizing method described below.

[Alkali solubilizing method]

To the cell-destructed solution was added sodium hydroxide so as to give a final concentration of 1 mol/L and the mixture was dissolved by heating (60°C, 30 minutes), then, the absorbance at 450 nm was measured using a micro plate reader. The melanine amount was calculated from a calibration curve made using synthetic melanine (SIGMA) as a standard. The melanine amount per unit protein was calculated by dividing the melanine amount by the protein amount.

(5) The melanine production suppressing rate was calculated according to the following formula.

$$\text{Melanine production suppressing rate (\%)} = [1-(A-B)/(C-B)]\times 100$$

[wherein, A represents the melanine amount per unit protein (g/g) in adding a sample, B represents the melanine amount per unit protein (g/g) in the normal group, and C represents the melanine amount per unit protein (g/g) in

the control group.]
In the above, Normal group is a case of theophylline (-; no addition) and sample (-; no addition), and Control group is a case of theophylline (+; addition) and sample (-; no addition).

(6) Determination of skin-lightening effect
The amount of a sample required for adjusting the melanogenesis inhibiting ratio to 40% or more was measured, and the effect was evaluated by the following criteria based on the measured amount. The measurement was conducted at N = 4 (4 wells per sample). The skin-lightening effect is expressed as described below based on the sample concentration showing the melanogenesis inhibiting ratio of 40% or more. The results are shown in Tables 1 to 3. The measurement was conducted at N = 4.

≥ 300 μmol/L : Δ
100 to 300 μmol/L : ○
30 to 100 μmol/L : ◎
≤ 30 μmol/L : ◎◎

[Table 1]

| Example No. | Sample | Judgment |
|---|---|---|
| Comparison | Kojic acid | Δ |
| Comparison | Ascorbic acid | Δ |
| Comparison | Arbutin | ◎ |
| Comparison | Hydroquinone | Δ |
| Comparison | 1-(4'-tert-butylphenoxy)propyl (Synthesis Example 1) | Δ |
| Comparison | 1-(4'-tert-butylphenoxy)ethyl (Synthesis Example 2) | Δ |
| Comparison | 1,2-di-hydroxy-3-phenoxypropyl (Synthesis Example 3) | Δ |
| Comparison | 1,2-di-hydroxy-3-(2'-methylphenoxy)propyl (Synthesis Example 4) | Δ |
| Example 1 | 1,2-di-hydroxy-3-(4'-tert-butylphenoxy) propyl | ◎◎ |
| Example 2 | 2-hydroxy-3-(4'-tert-butylphenoxy)propyl | ◎ |
| Example 3 | 1-O-ethyl-2-hydroxy-3-(4'-tert-butyl phenoxy)propyl | ◎◎ |
| Example 3 | 2-O-ethyl-1-hydroxy-3-(4'-tert-butyl phenoxy)propyl | ◎◎ |
| Example 4 | 1-O-ethyl-2-hydroxy-3-(4'-sec-butyl phenoxy)propyl | ◎ |
| Example 5 | 1,2-di-hydroxy-3-(4'-sec-butylphenoxy) propyl | ◎ |
| Example 6 | 1,2-di-hydroxy-3-(2'-sec-butylphenoxy) propyl | ○ |
| Example 7 | 1,2-di-octanoyl-3-(4'-tert-butylphenoxy) propyl | ◎◎ |
| Example 8 | 2-hydroxy-1,3-di-(4'-tert-butylphenoxy) propyl | ◎◎ |
| Example 9 | 1-(4" -hydroxyphenoxy)-2-hydroxy-3-(4'-tert-butylphenoxy)propyl | ◎ |

[Table 2]

| Example No. | Sample | Judgment |
|---|---|---|
| Example 10 | di[2-hydroxy-3-(4'-tert-butylphenoxy) propyl]ether | ○ |
| Example 11 | 1-0-cetyl-2-hydroxy-3-(4'-tert-butyl phenoxy)propyl | ○ |
| Example 12 | 1,2-di-hydroxy-3-(4'-n-butylphenoxy) propyl | ◎ |
| Example 13 | 1,2-di-hydroxy-3-(4'-isopropyl phenoxy)propyl | ◎ |
| Example 14 | 1,2-di-hydroxy-3-(4'-ethylphenoxy) propyl | ○ |

(continued)

| Example No. | Sample | Judgment |
|---|---|---|
| Example 15 | 1,2-di-hydroxy-3-(4'-tert-amyl phenoxy)propyl | ◎◎ |
| Example 16 | 1,2-di-hydroxy-3-(4'-α-cumylphenoxy) propyl | ◎ |
| Example 17 | 1,2-di-hydroxy-3-[4'-(1", 1", 3", 3" - tetramethylbutyl)phenoxy]propyl | ◎◎ |
| Example 18 | 1-hydroxy-3-(4'-tert-butylphenoxy) propyl | ◎ |
| Example 19 | 1,2-di-hydroxy-3-(2',6'-di-sec-butyl phenoxy)propyl | ◎◎ |
| Example 20 | 1,2-di-hydroxy-3-(2',4'-di-tert-butyl phenoxy)propyl | ◎◎ |
| Example 21 | 1,2-di-hydroxy-3-(2',4'-di-tert-butyl -5'-methylphenoxy)propyl | ◎◎ |
| Example 22 | 1,2-di-hydroxy-3-(2',6'-di-tert-butyl -4'-methylphenoxy)propyl | ◎◎ |
| Example 23 | 1,2-di-hydroxy-3-(2'-tert-butyl-4'-methoxylphenoxy)propyl | ◎ |

[Table 3]

| Example No. | Sample | Judgment |
|---|---|---|
| Example 24 | 1,2-di-hydroxy-3-(3'-tert-butyl-4'-methoxylphenoxy)propyl | ◎ |
| Example 25 | 2,2-bis[4'-(1", 2" -di-hydroxypropoxy) phenyl]propane | ◎◎ |
| Example 26 | 1-O-(2" -ethylhexyl)-2-hydroxy-3-(2'-sec-butylphenoxy)propyl | ◎ |
| Example 27 | 1-O-ethyl-2-hydroxy-3-(2'-sec-butyl phenoxy)propyl | ◎ |
| Example 28 | 1-O-ethyl-2-hydroxy-3-(4'-α-cumyl phenoxy)propyl | ◎◎ |
| Example 29 | 1-O-ethyl-2-hydroxy-3-(3'-methyl-4'-isoproylphenoxy)propyl | ◎◎ |
| Example 30 | 1-O-ethyl-2-hydroxy-3-(2',6'-di-sec-butylphenoxy)propyl | ◎◎ |
| Example 31 | 1,2-di-hydroxy-3-(3'-methyl-4'-isoproylphenoxy)propyl | ◎◎ |
| Example 32 | 2,2-bis[4'-(1" -O-ethyl-2" -hydroxy propoxy)phenyl]propane | ◎◎ |
| Example 33 | 1-O-ethyl-2-hydroxy-3-[4'-(1", 1", 3" ,3" -tetramethylbutyl)phenoxy]propyl | ◎ |

It is shown that the hydroxypropylalkylphenyl ether derivative of the present invention has a more excellent skin-lightening effect than known melanogenesis inhibitors, that is, kojic acid, arbutin and ascorbic acid. A lot of compound among the hydroxypropylalkylphenyl ether derivatives of the present invention exhibited an effect equivalent to or more than that of hydroquinone which is known as a substance showing a high skin-lightening effect and of which safety is a concern.

Test Example 2 Antimicrobial test

The propyl-phenyl-ether derivative of the present invention was evaluated for an antimicrobial test using four bacteria: Escherichia coli, Pseudomonas aeruginosa, Staphylococcus aureus and Candida albicans (use of ATCC strain) according to the following procedure. As a comparative compound, 1,2-pentandiol was used.

(1) Into a test tube containing 1.0 g of Tween 80 (polyoxyethylene sorbitan monooleate) added, 0.3 g, 0.1 g or 0.05 g of various samples were added, and SCD (Soybean Casein Digest) agar medium was added each in an amount of about 10 mL so as to give final concentrations of 3.0%, 1.0% and 0.5%, and stirred thoroughly with Vortex. Thereafter, the sample-containing medium was sterilized by high-pressure steam sterilization (for 15 minutes at 121°C), cooled down to 45°C, the medium was poured gently onto a petri dish sterilized previously, and spread over the whole petri dish having signage of 4-section on the outer surface of the bottom, and allowed to stand still to solidify the medium.

(2) Thereafter, various bacterium solutions of which number of bacteria had been previously regulated to $1.0 \times 10^4$

to $5.0 \times 10^4$ cfu/mL were inoculated on the sample-containing medium prepared in (1), each one bacterium for one section of the petri dish, each in an amount of 10 μL on four places (three concentrations are applied to four kinds of bacteria for each sample, that is, 12 specimens per sample).
(3) These were cultured in a 30°C thermostat bath for 7 days.
(4) The presence or absence of bacteria was determined visually.

The results are classified by the following criteria and shown in Table 4.

- The number of specimens confirming bacteria was 10 or more: Δ
- The number of specimens confirming bacteria was 6 to 9: ○
- The number of specimens confirming bacteria was 5 or less: ◎

(Table 4)

| Sample | Sample | judgment |
|---|---|---|
| Comparative compound | 1,2-pentandiol | Δ |
| Example 1 | 1,2-dihydroxy-3-(4'-tert-butyl phenoxy)propyl | ◎ |
| Example 3 | 1-O-ethyl-2-hydroxy-3-(4'-tert-butyl phenoxy)propyl | ◎ |
| Example 5 | 1,2-di-hydroxy-3-(4'-sec-butyl phenoxy)propyl | ◎ |
| Example 6 | 1,2-di-hydroxy-3-(2'-sec-butyl phenoxy)propyl | ○ |
| Example 7 | 1,2-di-octanoyl-3-(4'-tert-butylphenoxy)propyl | ○ |

Test Example 3 Anti-acne test

Propionibacterium acnes (JCM6415: ATCC strain) was transplanted to GAM bouillon medium (manufactured by Nissui Pharmaceutical Co., Ltd.), and cultured at 37°C under anaerobic condition for 24 hours. Thereafter, it was diluted with GAM bouillon medium so that the final bacterium concentration was $OD_{620}$ = 0.1, and this was used as a test bacterium solution. In examples, the propyl-phenyl-ether derivative was weighed so that the final concentration was 0.1%, dissolved in GAM bouillon medium, then, filter-sterilization was performed. The resultant solution was used as a sample solution. The test bacterium solution was added in an amount of 30 μL and the sample solution was added in an amount of 120 μL to a 96-well microplate (manufactured by Sumitomo Bakelite Co., Ltd.), then, immediately, the micro plate and AnaeroPack-KENKI (manufactured by Mitsubishi Gas Chemical Co., Inc.) were placed into an anaerobic jar (manufactured by Mitsubishi Gas Chemical Co., Inc.), and culture was carried out at 37°C under anaerobic condition for 48 hours. Thereafter, $OD_{620}$ was measured by a microplate reader. The same test was conducted using methylparaben, phenoxyethanol and sodium ascorbate as comparative compounds.
The results are classified by the following criteria and shown in Table 5 - Table 7.

- Δ : $OD_{620}$ is 0.6 or more
- ○ : $OD_{620}$ is 0.3 - 0.6
- ◎ : $OD_{620}$ is 0.3 or less

[Table 5]

| Example No. | Sample Compound | Judgment |
|---|---|---|
| Comparison | Methylparaben | Δ |
| Comparison | Phenoxyethanol | Δ |
| Comparison | Sodium Ascorbate | Δ |
| Comparison | 1-(4'-tert-butylphenoxy)propyl (Synthesis Example 1) | Δ |
| Comparison | 1-(4'-tert-butylphenoxy)ethyl (Synthesis Example 2) | Δ |
| Comparison | 1,2-di-hydroxy-3-phenoxypropyl (Synthesis Example 3) | Δ |

(continued)

| Example No. | Sample Compound | Judgment |
|---|---|---|
| Comparison | 1,2-di-hydroxy-3-(2'-methylphenoxy)propyl (Synthesis Example 4) | Δ |
| Example 1 | 1,2-di-hydroxy-3-(4'-tert-butylphenoxy) propyl | ◎◎ |
| Example 2 | 2-hydroxy-3-(4'-tert-butylphenoxy)propyl | ◎ |
| Example 3 | 1-O-ethyl-2-hydroxy-3-(4'-tert-butyl phenoxy)propyl | ○ |
| Example 3 | 2-O-ethyl-1-hydroxy-3-(4'-tert-butyl phenoxy)propyl | ◎◎ |
| Example 4 | 1-O-ethyl-2-hydroxy-3-(4'-sec-butyl phenoxy)propyl | ◎ |
| Example 5 | 1,2-di-hydroxy-3-(4'-sec-butylphenoxy) propyl | ○ |
| Example 6 | 1,2-di-hydroxy-3-(2'-sec-butylphenoxy) propyl | ○ |
| Example 7 | 1,2-di-octanoyl-3-(4'-tert-butylphenoxy) propyl | ○ |
| Example 8 | 2-hydroxy-1,3-di-(4'-tert-butylphenoxy) propyl | ◎◎ |
| Example 9 | 1-(4" -hydroxyphenoxy)-2-hydroxy-3-(4'-tert-butylphenoxy)propyl | ◎ |

[Table 6]

| Example No. | Sample Compound | Judgment |
|---|---|---|
| Example 10 | di[2-hydroxy-3-(4'-tert-butylphenoxy) propyl]ether | ◎ |
| Example 11 | 1-O-cetyl-2-hydroxy-3-(4'-tert-butyl phenoxy)propyl | ◎ |
| Example 12 | 1,2-di-hydroxy-3-(4'-n-butylphenoxy) propyl | ○ |
| Example 13 | 1,2-di-hydroxy-3-(4'-isopropyl phenoxy)propyl | ◎ |
| Example 14 | 1,2-di-hydroxy-3-(4'-ethylphenoxy) propyl | ◎ |
| Example 15 | 1,2-di-hydroxy-3-(4'-tert-amyl phenoxy)propyl | ◎◎ |
| Example 16 | 1,2-di-hydroxy-3-(4'-α-cumylphenoxy) propyl | ◎ |
| Example 17 | 1,2-di-hydroxy-3-[4'-(1", 1", 3", 3" - tetramethylbutyl)phenoxy]propyl | ◎◎ |
| Example 18 | 1-hydroxy-3-(4'-tert-butylphenoxy) propyl | ◎ |
| Example 19 | 1,2-di-hydroxy-3-(2',6'-di-sec-butyl phenoxy)propyl | ◎◎ |
| Example 20 | 1,2-di-hydroxy-3-(2',4'-di-tert-butyl phenoxy)propyl | ◎◎ |
| Example 21 | 1,2-di-hydroxy-3-(2',4'-di-tert-butyl -5'-methylphenoxy)propyl | ◎◎ |
| Example 22 | 1,2-di-hydroxy-3-(2',6'-di-tert-butyl -4'-methylphenoxy)propyl | ◎◎ |
| Example 23 | 1,2-di-hydroxy-3-(2'-tert-butyl-4'-methoxylphenoxy)propyl | ◎ |
| Example 24 | 1,2-di-hydroxy-3-(3'-tert-butyl-4'-methoxylphenoxy)propyl | ◎ |

[Table 7]

| Example No. | Sample Compound | Judgment |
|---|---|---|
| Example 25 | 2'2-bis[4'-(1", 2" -di-hydroxypropoxy) phenyl]propane | ◎◎ |
| Example 26 | 1-O-(2" -ethylhexyl)-2-hydroxy-3-(2'-sec-butylphenoxy)propyl | ◎ |
| Example 27 | 1-O-ethyl-2-hydroxy-3-(2'-sec-butyl phenoxy)propyl | ◎ |
| Example 28 | 1-O-ethyl-2-hydroxy-3-(4'-α-cumyl phenoxy)propyl | ◎◎ |
| Example 29 | 1-O-ethyl-2-hydroxy-3-(3'-methyl-4'-isoproylphenoxy)propyl | ◎◎ |

(continued)

| Example No. | Sample Compound | Judgment |
|---|---|---|
| Example 30 | 1-O-ethyl-2-hydroxy-3-(2',6'-di-sec-butylphenoxy)propyl | ◎◎ |
| Example 31 | 1,2-di-hydroxy-3-(3'-methyl-4'-isoproylphenoxy)propyl | ◎◎ |
| Example 32 | 2, 2-bis[4'-(1"-O-ethyl-2" -hydroxy propoxy)phenyl]propane | ◎◎ |
| Example 33 | 1-O-ethyl-2-hydroxy-3-[4'-(1", 1", 3" ,3" -tetramethylbutyl)phenoxy]propyl | ◎ |

Test Example 4 [Stability test]

Stability of coloration when 1,2-di-hydroxy-3-(4'-tert-butylphenoxy)propyl in Example 1 was stored at 50°C for 4 weeks was evaluated as described below. Aascorbic acid, kojic acid, hydroquinone and arbutin which are known skin-lightening agents were used as comparative compounds.

The test samples were added to a 50% ethanol solution so as to give a concentration of 1%, and pH of each was adjusted to 6.0 to 8.0 with a dilute sodium hydroxide aqueous solution or a dilute hydrochloric acid aqueous solution. The solutions were placed in 50 mL screw tubes and closely sealed, and stored at 50°C for 4 weeks. The degrees of coloration of the solution directly after preparation of the test sample, 2 weeks after storage and 4 weeks after storage were evaluated by 10 panelists according to the following criteria.

3: almost no change as compared with directly after preparation
2: colored as compared with directly after preparation
1: intensely colored as compared with directly after preparation

The evaluation results of coloration were classified as described below. The results are shown in Table 8.

○: total points of 10 panelists is 22 or more
Δ: total points of 10 panelists is 15 to 21
×: total points of 10 panelists is 14 or less

(Table 8)

| reagent | 2 weeks after | 4 weeks after |
|---|---|---|
| Ascorbic acid (comparative compound) | × | × |
| Kojic acid (comparative compound) | ○ | Δ |
| Hydroquinone (comparative compound) | × | × |
| Arbutin (comparative compound) | ○ | Δ |
| 1,2-di-hydroxy-3-(4'-tert-butylphenoxy)propyl (Example 1) | ○ | ○ |

As shown in Table 8, it was confirmed that 1,2-di-hydroxy-3-(4'-tert-butylphenoxy)propyl of the present invention was excellent in stability in terms of coloration.

Example 34 Cream

Oil phase part raw materials No. 1 to 6 and aqueous phase part raw materials No. 7 to 10 having compositions shown in Table 9 were heated up to 70°C and dissolved, to prepare an oil phase and an aqueous phase, respectively. Thereafter, the oil phase was added to the aqueous phase. The mixture is pre-emulsified, and emulsified uniformly by a homo-mixer. Then, the mixture is cooled down to room temperature while stirring thoroughly, to prepare a cream which can be considered excellent in skin-lightening effect. In tables of Table 9 or later, the compounding amount shows part by mass.

[Table 9]

| No | Name of component | Compounding amount |
|---|---|---|
| 1 | Squalane | 8.0 |
| 2 | Vaseline | 5.0 |
| 3 | Steary alcohol | 5.0 |
| 4 | Polyoxyethylene(25) cetyl ether | 2.5 |
| 5 | Glyceryl monostearate | 1.5 |
| 6 | 1-O-ethyl-2-hydroxy-3-(4'-tert-butylphenoxy)propyl (obtained in Example 3) | 1.0 |
| 7 | Glycerin | 5.0 |
| 8 | Antiseptic | proper amount |
| 9 | pH regulator | proper amount |
| 10 | Purified water | Residual amount |

Example 35 Milky Lotion

Oil phase part raw materials No. 1 to 10 and aqueous phase part raw materials No. 11 to 13 having compositions shown in Table 10 were heated up to 70°C and dissolved, to prepare an oil phase and an aqueous phase, respectively. Thereafter, the oil phase was added to the aqueous phase. The mixture is pre-emulsified, and emulsified uniformly by a homo-mixer, then, cooled down to room temperature while stirring thoroughly, to prepare a milky lotion which can be considered excellent in skin-lightening effect.

[Table 10]

| No | Name of component | Compounding amount |
|---|---|---|
| 1 | Isosteary palmitate | 4.0 |
| 2 | Jojoba oil | 1.0 |
| 3 | Dimethylpolysiloxane | 2.0 |
| 4 | Cetanol | 1.0 |
| 5 | Stearic acid | 1.5 |
| 6 | Bees wax | 2.5 |
| 7 | Paraffin wax | 2.5 |
| 8 | Polyoxyethylene(20) sorbitan monostearate | 1.2 |
| 9 | Polyoxyethylene(40) sorbitol tetraoleate | 1.5 |
| 10 | 2-hydroxy-3-(4'-tert-butylphenoxy) propyl (obtained in Example 2) | 10.0 |
| 11 | Propylene glycol | 3.0 |
| 12 | Antiseptic | proper amount |
| 13 | Purified water | Residual amount |

Example 36 Milky Lotion

Oil phase part raw materials No. 4 to 10 and aqueous phase part raw materials No. 1 to 3 and 11 to 12 having compositions shown in Table 11 were heated up to 70°C and dissolved, to prepare an oil phase and an aqueous phase, respectively. Thereafter, the oil phase was added to the aqueous phase. The mixture is pre-emulsified, and emulsified uniformly by a homo-mixer, then, cooled down to room temperature while stirring thoroughly, to prepare a milky lotion which can be considered excellent in skin-lightening effect.

[Table 11]

| No | Name of component | Compounding amount |
|---|---|---|
| 1 | Dipropylene glycol | 3.0 |
| 2 | Sorbitansesqui oleate | 3.0 |
| 3 | Polyoxyethylene(20) sorbitan monooleate | 1.0 |
| 4 | 1,2-di-hydroxy-3-(4'-sec-butylphenoxy) propyl (obtained in Example 5) | 5.0 |
| 5 | Micro crystalline wax | 1.5 |
| 6 | Bees wax | 2.5 |
| 7 | Lanolin | 2.0 |
| 8 | Liquid paraffin | 16.5 |
| 9 | Squalane | 10.0 |
| 10 | Perfume | proper amount |
| 11 | Antiseptic | proper amount |
| 12 | Purified water | Residual amount |

Example 37 Cream

Oil phase part raw materials No. 1 to 3 and aqueous phase part raw materials No. 4 to 10 having compositions shown in Table 12 were heated up to 70°C and dissolved, to prepare an oil phase and an aqueous phase, respectively. Thereafter, the oil phase was added to the aqueous phase. The mixture is pre-emulsified, and emulsified uniformly by a homo-mixer, then, cooled down to room temperature while stirring thoroughly, to prepare a cream which can be considered excellent in skin-lightening effect.

[Table 12]

| No | Name of component | Compounding amount |
|---|---|---|
| 1 | Liquid paraffin | 15.0 |
| 2 | Vaseline | 15.0 |
| 3 | 1,2-di-hydroxy-3-(2'-sec-butylphenoxy) propyl (obtained in Example 6) | 5.0 |
| 4 | Carboxyvinylpolymer | 0.1 |
| 5 | Xanthan gum | 0.1 |
| 6 | Hardened castor oil polyoxyethylene(40) derivative | 3.0 |
| 7 | Sodium hydroxide | 0.05 |
| 8 | Perfume | proper amount |
| 9 | Antiseptic | proper amount |
| 10 | Purified water | Residual amount |

Example 38 Cream

Oil phase part raw materials No. 1 to 6 and aqueous phase part raw materials No. 7 to 10 having compositions shown in Table 13 were heated up to 70°C and dissolved. The oil phase was added to the aqueous phase. The mixture was pre-emulsified, and emulsified by a homo-mixer, then, cooled down to room temperature while stirring thoroughly, to prepare a cream which can be considered excellent in skin-lightening effect.

[Table 13]

| No | Name of component | Compounding amount |
|---|---|---|
| 1 | Cetylalcohol | 2.0 |
| 2 | Steary alcohol | 3.0 |
| 3 | Squalane | 6.5 |
| 4 | Glyceryl tri-2-ethylhexanate | 5.5 |
| 5 | Methyl Polysiloxane | 5.5 |
| 6 | di[2-hydroxy-3-(4'-tert-butylphenoxy) propyl]ether (obtained in Example 10) | 1.0 |
| 7 | 1,3-Butylene glycol | 5.0 |
| 8 | Hydroxyethyl cellulose | 0.2 |
| 9 | Antiseptic | proper amount |
| 10 | Purified water | Residual amount |

Example 39 Lotion

A lotion was prepared by mixing raw materials No. 1 to 6 having compositions shown in Table 14 while stirring thoroughly. Since this lotion contains hydroxylpropyl-phenyl-ether derivative, it can be considered that this lotion is excellent in skin-lightening effect and can be used as a skin-lightening cosmetic.

[Table 14]

| No | Name of component | Compounding amount |
|---|---|---|
| 1 | 2-hydroxy-1,3-di-(4'-tert-butyl phenoxy)propyl (obtained in Example 8) | 0.05 |
| 2 | ethanol | 10.0 |
| 3 | Citric acid | 0.01 |
| 4 | Sodium citrate | 0.015 |
| 5 | Potassium glycyrrhizinate | 0.03 |
| 6 | Purified water | Residual amount |

## Claims

**1.** A propyl-phenyl-ether derivative represented by the following general formula (I), (Ia) or (Ib):

(Chemical formula 1)

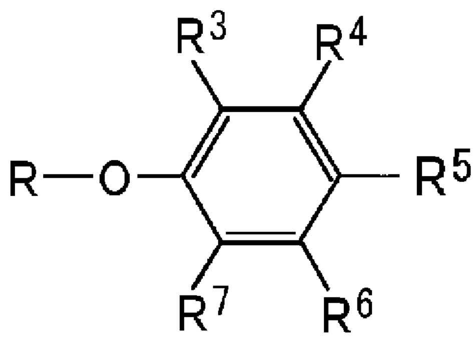

(I)

(Chemical formula 2)

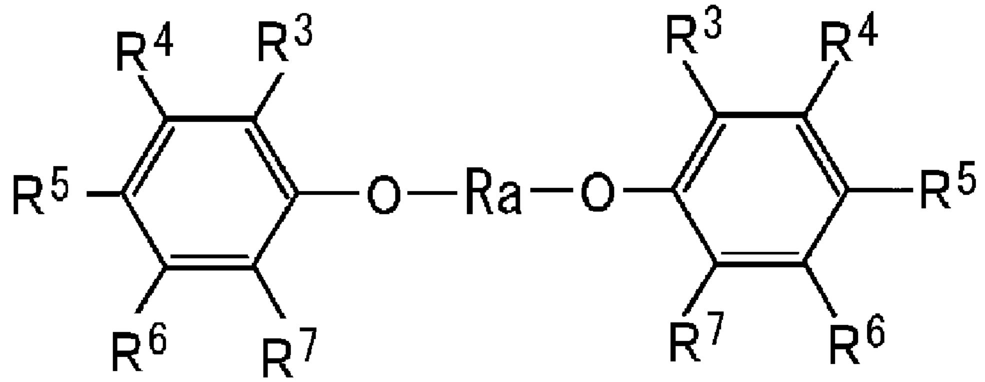

(Ia)

(Chemical formula 3)

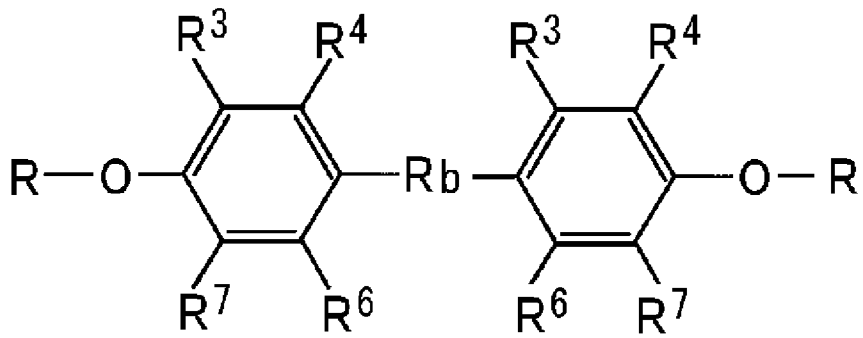

(Ib)

[In the formula (I), (Ia) or (Ib),

R represents $-CH_2-CHR^2-CH_2R^1$ or $-CH(CH_2R^2)-CH_2R^1$,
Ra represents $-CH_2-CHR^2-CH_2-O-CH_2-CHR_2-CH_2-$ or $-CH_2-CHOH-CH_2-$,
Rb represents a linear or branched aliphatic hydrocarbon group having 1 to 5 carbon atoms,
$R^1$ and $R^2$ represent each independently hydrogen, $R^8O$, $R^9S$, $R^{10}R^{11}N$, $R^{12}A1$, a linear or branched, saturated or unsaturated aliphatic hydrocarbon group having 1 to 22 carbon atoms, or a saturated or unsaturated cyclic hydrocarbon group having 3 to 22 carbon atoms,
$R^8$, $R^9$, $R^{10}$, $R^{11}$ and $R^{12}$ represent each independently a linear or branched, saturated or unsaturated aliphatic hydrocarbon group having 1 to 22 carbon atoms, a saturated or unsaturated cyclic hydrocarbon group having 3 to 22 carbon atoms of which hydrogen may be substituted with a hydroxyl group, or hydrogen,
A1 represents a phosphate group, a sulfate group, an ester group, a thioester group or an amide group or a salt thereof, and here,
at least one of $R^1$ or $R^2$ is a hydroxyl group, or a group represented by $R^{12}A1$- in which A is a phosphate group, a sulfate group or an ester group or a salt thereof, and
$R^3$, $R^4$, $R^5$, $R^6$ and $R^7$ represent each independently
a linear or branched, saturated or unsaturated aliphatic hydrocarbon group having 1 to 12 carbon atoms of which hydrogen may be substituted with a phenyl group, an alkoxy group having 1 to 5 carbon atoms, a phenyl group, or hydrogen,
and when both $R^1$ and $R^2$ are a group selected from the group consisting of a hydroxyl group and a group represented by $R^{12}$ A1- in which A is a phosphate group, a sulfate group or an ester group or a salt thereof, the sum of the number of carbon atoms of $R^3$, $R^4$, $R^5$, $R^6$ and $R^7$ is 2 or more].

**2.** A propyl-phenyl-ether derivative according to Claim 1, in which $R^1$ or/and $R^2$ are a group selected from hydrogen, a linear or branched, saturated or unsaturated aliphatic hydrocarbon group having 1 to 22 carbon atoms, $R^8O$, $R^9S$ and $R^{10}R^{11}N$.

**3.** A propyl-phenyl-ether derivative according to Claim 2, in which $R^1$ is $R^8O$ and $R^2$ is a hydroxyl group.

**4.** A propyl-phenyl-ether derivative according to Claim 3, which is represented by the general formula (I).

**5.** A propyl-phenyl-ether derivative according to Claim 4, in which $R^8$ is hydrogen or a linear or branched, saturated or unsaturated aliphatic hydrocarbon group having 1 to 22 carbon atoms.

**6.** A propyl-phenyl-ether derivative according to Claim 5, in which $R^8$ is a linear or branched, saturated or unsaturated aliphatic hydrocarbon group having 1 to 22 carbon atoms.

**7.** A propyl-phenyl-ether derivative according to any one of Claims 1 - 6, which is represented by the general formula (I) in which $R^3$, $R^4$, $R^5$, $R^6$ and $R^7$ represent each independently hydrogen or a linear or branched, saturated or unsaturated aliphatic hydrocarbon having 1 to 12 carbon atoms.

**8.** A propyl-phenyl-ether derivative according to Claim 7, in which at least one group among $R^3$, $R^4$, $R^5$, $R^6$ and $R^7$ is a branched aliphatic hydrocarbon and the sum of the number of carbon atoms of $R^3$, $R^4$, $R^5$, $R^6$ and $R^7$ is 4 or more.

**9.** A propyl-phenyl-ether derivative according to Claim 8, in which at least one group among $R^3$, $R^4$, $R^5$, $R^6$ and $R^7$ is a tert-butyl group or a sec-butyl group.

**10.** A propyl-phenyl-ether derivative according to Claim 1, which is represented by the general formula (Ia) in which Ra is $-CH_2-CH(OH)-CH_2-$.

**11.** A propyl-phenyl-ether derivative according to Claim 1, which is represented by the general formula (Ib) in which Rb is $-C(CH_3)_2-$.

**12.** A propyl-phenyl-ether derivative which is represented by the following structural formula (II):

(Chemical formula 4)

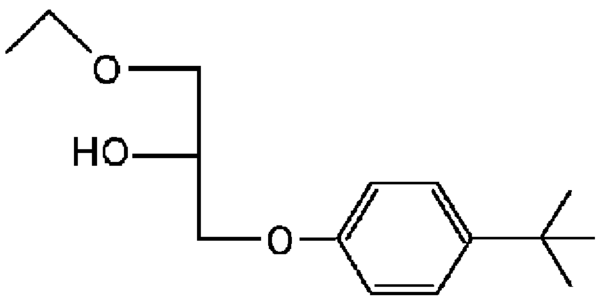

(II)

.

**13.** A propyl-phenyl-ether derivative which is represented by the following structural formula (A):

(Chemical formula 5)

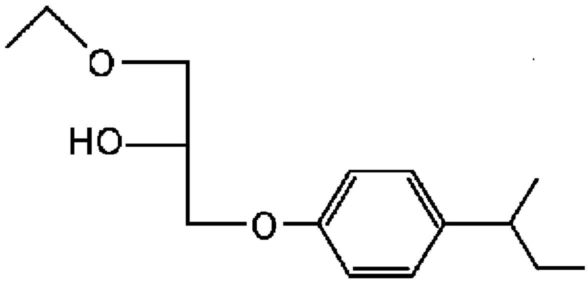

(A)

**14.** A propyl-phenyl-ether derivative which is represented by the following structural formula (C):

(Chemical formula 6)

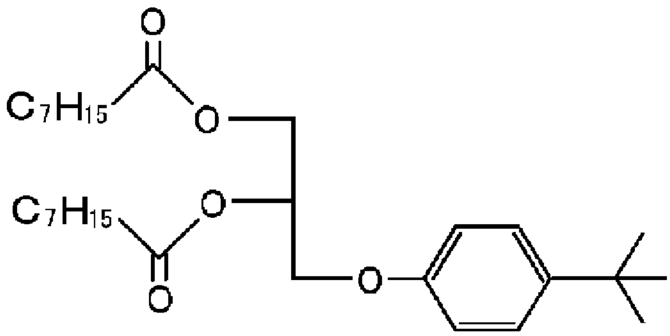

(C)

**15.** A propyl-phenyl-ether derivative which is represented by the following structural formula (E):

(Chemical formula 7)

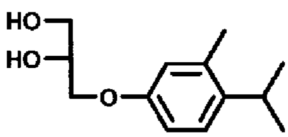

(E)

**16.** A propyl-phenyl-ether derivative which is represented by the following structural formula (F):

(Chemical formula 8)

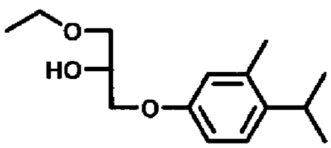

(F)

**17.** A propyl-phenyl-ether derivative which is represented by the following structural formula (G):

(Chemical formula 9)

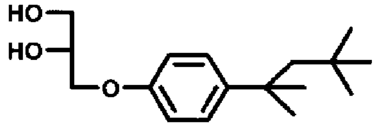 (G)

**18.** A propyl-phenyl-ether derivative which is represented by the following structural formula (H):

(Chemical formula 10)

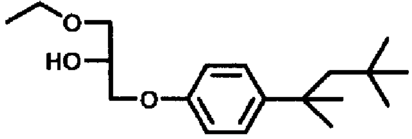 (H)

**19.** A propyl-phenyl-ether derivative which is represented by the following structural formula (P):

(Chemical formula 11)

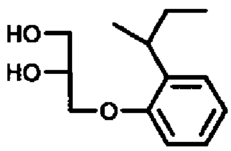 (P)

**20.** A propyl-phenyl-ether derivative which is represented by the following structural formula (J):

(Chemical formula 12)

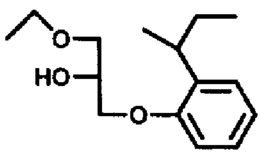 (J)

**21.** A propyl-phenyl-ether derivative which is represented by the following structural formula (K):

(Chemical formula 13)

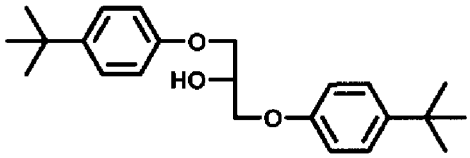

(K)

.

22. A melanogenesis inhibitor which is prepared by formulating the propyl-phenyl-ether derivative according to any one of Claims 1 to 21 as an active ingredient.

23. A melanogenesis inhibitor which is prepared by formulating the propyl-phenyl-ether derivative represented by the following formula (III) as an active ingredient:

(Chemical formula 14)

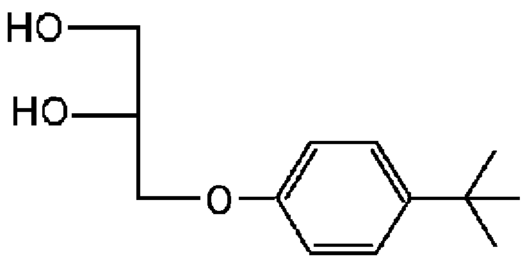

(III)

.

24. A melanogenesis inhibitor which is prepared by formulating the propyl-phenyl-ether derivative represented by the following formula (D) as an active ingredient:

(Chemical formula 15)

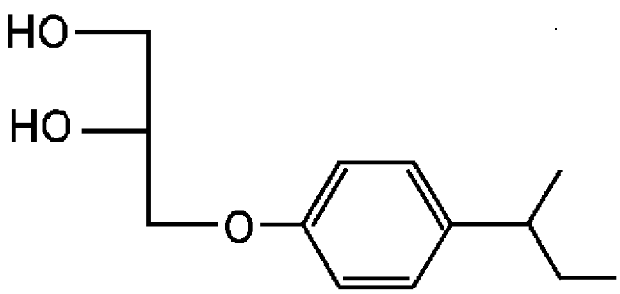

(D)

.

25. A skin-lightening agent which is prepared by formulating the propyl-phenyl-ether derivative according to any one of Claims 1 to 21 as an active ingredient.

26. An antimicrobial agent which is prepared by formulating the propyl-phenyl-ether derivative according to any one of Claims 1 to 21 as an active ingredient.

**27.** A cosmetic which is prepared by formulating the propyl-phenyl-ether derivative compound and its salt according to any one of Claims 1 to 21 as an active ingredient.

**INTERNATIONAL SEARCH REPORT**

International application No.
PCT/JP2013/079498

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY/CASREACT(STN)

C. DOCUMENTS CONSIDERED TO BE RELEVANT

| Category* | Citation of document, with indication, where appropriate, of the relevant passages | Relevant to claim No. |
|---|---|---|
| X<br>A | YU, T. et al., 3-(4-(tert-Octyl)phenoxy) propane-1,2-diol suppresses inflammatory responses via inhibition of multiple kinases, Biochemical Pharmacology, 83(11), 2012.06.01, p.1540-1551 | 1-5,7,8,10, 17,22,25-27<br>6,9,11-16, 18-21,23,24 |
| X<br>A | HALL, L. H. et al., Molecular connectivity and substructure analysis, Journal of Pharmaceutical Sciences, 67(12), 1978, p.1743-1747 | 1,2,7,26,27<br>3-6,8-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 January, 2014 (14.01.14) | 28 January, 2014 (28.01.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No. PCT/JP2013/079498

C (Continuation). DOCUMENTS CONSIDERED TO BE RELEVANT

| Category* | Citation of document, with indication, where appropriate, of the relevant passages | Relevant to claim No. |
|---|---|---|
| X<br>A | YEH, J. L. et al., Eugenolol and glyceryl-isoeugenol suppress LPS-induced iNOS expression by down-regulating NF-κB and AP-1 through inhibition of MAPKS and AKT/IκBα signaling pathways in macrophages, International Journal of Immunopathology and Pharmacology, 24(2), 2011, p.345-356 | 1-5,26,27<br>6-25 |
| X<br>A | BERGER, F. M. et al., Antimicrobial action of certain glycerol ethers and related compounds, Applied Microbiology, 1, 1953, p.146-149 | 1,2,7,26,27<br>3-6,8-25 |
| X<br>A | OSANAI, S. et al., Synthesis of alkoxyl and phenoxyl substituted glycerides and their antimicrobial properties, Bokin Bobai, 14(3), 1986, p.109-116 | 1,2,7,26,27<br>3-6,8-25 |
| X<br>A | AUZOU, G. et al., Anxiolytic activity of tert-butyl phenol derivatives. II. Dioxolanones, European Journal of Medicinal Chemistry, 10(1), 1975, p.89-90 | 1-5,7-9<br>6,10-27 |
| X<br>A | THEIL, F. et al., Kinetic resolution of acyclic 1,2-diols using a sequential lipase-catalyzed transesterification in organic solvents, Journal of Organic Chemistry, 59(2), 1994, p.388-393 | 1-5,7-9,14<br>6,10-13,<br>15-27 |
| X<br>A | THEIL, F. et al., Lipase-catalyzed resolution of 3-(aryloxy)-1,2-propanediol derivatives. Towards an improved active site model of Pseudomonas cepacia lipase (Amano PS), Tetrahedron: Asymmetry, 6(6), 1995, p.1323-1344 | 1-5,7-10,14<br>6,11-13,<br>15-27 |
| X<br>A | JP 7-84350 A (Fuji Photo Film Co., Ltd.), 31 March 1995 (31.03.1995), paragraph [0035]; examples & US 5468600 A | 1-5,7-10<br>6,11-27 |
| X<br>A | AUZOU, G. et al., Anxiolytic activity derived from a myorelaxant structure. tert-Butylphenol derivatives, European Journal of Medicinal Chemistry, 9(5), 1974, p.548-554 | 1-3,10,21<br>4-9,11-20,<br>22-27 |
| X<br>A | US 3717611 A (WILBELM Baumer), 20 February 1973 (20.02.1973), 8th row, lines 25, 35; examples & GB 1251625 A | 1-10<br>11-27 |
| X<br>A | JP 2011-526250 A (British Columbia Cancer Agency Branch), 06 October 2011 (06.10.2011), claim 25 (Family: none) | 1-3,11<br>4-10,12-27 |

Form PCT/ISA/210 (continuation of second sheet) (July 2009)

**INTERNATIONAL SEARCH REPORT**

International application No.
PCT/JP2013/079498

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 8-54716 A (Konica Corp.),<br>27 February 1996 (27.02.1996),<br>paragraph [0054]<br>& US 5576161 A & EP 697625 A2 | 1,2<br>3-27 |
| A | JP 63-246311 A (Shiseido Co., Ltd.),<br>13 October 1988 (13.10.1988),<br>synthesis examples; examples<br>(Family: none) | 1-27 |

Form PCT/ISA/210 (continuation of second sheet) (July 2009)

**INTERNATIONAL SEARCH REPORT**

International application No.
PCT/JP2013/079498

Continuation of A. CLASSIFICATION OF SUBJECT MATTER
(International Patent Classification (IPC))

*C07C43/23*(2006.01)i, *A01N31/14*(2006.01)i, *A01P3/00*(2006.01)i, *A61K8/34*(2006.01)i, *A61K8/37*(2006.01)i, *A61K8/40*(2006.01)i, *A61K8/41*(2006.01)i, *A61K8/44*(2006.01)i, *A61K8/46*(2006.01)i, *A61K31/085*(2006.01)i, *A61K31/10*(2006.01)i, *A61K31/136*(2006.01)i, *A61K31/192*(2006.01)i, *A61K31/198*(2006.01)i, *A61K31/277*(2006.01)i, *A61P17/00*(2006.01)i, *A61P31/04*(2006.01)i, *A61P43/00*(2006.01)i, *A61Q19/00*(2006.01)i, *A61Q19/02*(2006.01)i, *C07C65/21*(2006.01)i, *C07C205/37*(2006.01)i, *C07C217/30*(2006.01)i, *C07C217/84*(2006.01)i, *C07C229/26*(2006.01)i, *C07C255/54*(2006.01)i, *C07C279/14*(2006.01)i, *C07C323/12*(2006.01)i

(According to International Patent Classification (IPC) or to both national classification and IPC)

Continuation of B. FIELDS SEARCHED
Minimum documentation searched (International Patent Classification (IPC))

C07C43/23, A01N31/14, A01P3/00, A61K8/34, A61K8/37, A61K8/40, A61K8/41, A61K8/44, A61K8/46, A61K31/085, A61K31/10, A61K31/136, A61K31/192, A61K31/198, A61K31/277, A61P17/00, A61P31/04, A61P43/00, A61Q19/00, A61Q19/02, C07C65/21, C07C205/37, C07C217/30, C07C217/84, C07C229/26, C07C255/54, C07C279/14, C07C323/12

Minimum documentation searched (classification system followed by classification symbols)

Form PCT/ISA/210 (extra sheet) (July 2009)

**INTERNATIONAL SEARCH REPORT**

International application No.
PCT/JP2013/079498

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention described in claim 1 includes inventions respectively relating to multiple compounds included within the scope of the chemical structural formulae represented by general formulae (I), (Ia) and (Ib). However, the inventions are known, as disclosed in the documents cited in the international search report and the like. Therefore, the inventions respectively relating to the compounds recited in claim 1 do not comply with the requirement of unity of invention as set forth in PCT Rule 13.2.

The above-said opinion may be also applied to the inventions of claims 2-27 in the present application.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invention.

☐ No protest accompanied the payment of additional search fees.

Form PCT/ISA/210 (continuation of first sheet (2)) (July 2009)

**INTERNATIONAL SEARCH REPORT**

International application No.
PCT/JP2013/079498

The invention described in claim 1 of the present application relates to propyl phenyl ether derivatives respectively represented by general formulae (I), (Ia) and (Ib), and includes compounds having various backbones and various substituents.

However, those compounds which are disclosed in the meaning within PCT Article 5 are only compounds having specific chemical structures which are mentioned specifically and of which the production methods and properties are also mentioned specifically in the section "examples".

Therefore, the invention of claim 1 in the present application are not fully supported within the meaning of PCT Article 6.

Such being the case, with respect to claim 1 of the present application, the search was carried out on compounds which are expressed by alternatives that can be specified by the compounds mentioned as examples.

The above-said opinion may be also applied to the inventions of claims 2-11, 22 and 25-27 in the present application.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6192062 A
- JP 4658898 B
- JP 3340930 B
- JP 4828126 B

### Non-patent literature cited in the description

- *Journal of Investigative Dermatology,* 2000, vol. 114 (1), 157-164